# EUROPEAN PATENT APPLICATION

(11) **EP 1 764 099 A2**
(43) Date of publication of application: **21.03.2007**
(21) Application number: 06120683.5
(22) Date of filing: 14.09.2006
(51) Int. Cl.: A61K 31/404, A61P 25/28, A61P 31/18, A61P 33/06

(54) **Diaminoalcohol derivatives for the treatment of Alzheimer, malaria, HIV**

(30) Priority: 17.09.2005 CH 15232005
(71) Applicant: Speedel Experimenta AG, 4123 Allschwil (CH)
(72) Inventor: Herold, Peter, 4123, Allschwil (CH); Mah, Robert, 4123, Allschwil (CH); Stutz, Stefan, 4123, Allschwil (CH); Tschinke, Vincenzo, 4123, Allschwil (CH); Stojanovic, Aleksandar, 4123, Allschwil (CH); Marti, Christiane, 4123, Allschwil (CH); Behnke, Dirk, 4123, Allschwil (CH); Jotterand, Nathalie, 4123, Allschwil (CH); Quirmbach, Michael, 4123, Allschwil (CH); Schumacher, Christoph, 4123, Allschwil (CH)
(74) Representative: Maué, Paul Georg

(57) **Abstract**

Use of compounds of the general formula (I) 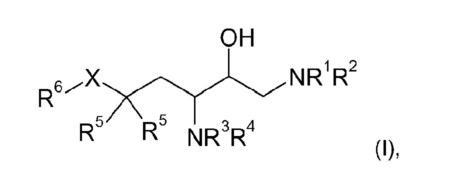 wherein R¹, R², R³, R⁴, R⁵, R⁶ and X have the definitions elucidated in more detail in the description, as beta-secretase, cathepsin D, plasmepsin II and/or HIV protease inhibitors.

## Description

The present invention relates to the use of aminoalcohols as beta-secretase-, cathepsin D-, plasmepsin II- and/or HIV-protease-inhibitors.

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias.

### Alzheimner's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity.

As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data detailing both the catalytic mechanism and the molecular basis for substrate selection.

Firstly, the present invention relates to the use as beta-secretase-, cathepsin D-, plasmepsin II- and/or HIV-protease-inhibitors of compounds of the general formula 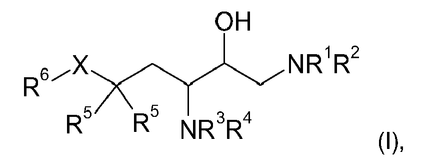 or a pharmaceutically usable salt thereof; wherein
X is methylene or hydroxymethylene;
R¹
a) is hydrogen; or
b) is C₁-C₈-alkyl, C₃-C₈-cydoalkyl, C₁-C₈-alkanoyl, C₁-C₈-alkoxycarbonyl, aryl-C₀-C₄-alkyl or heterocyclyl-C₀-C₄-alkyl, which radicals may be substituted by 1-4 C₁-C₈-alkyl, halogen, cyano, oxide, oxo, trifluoromethyl, C₁-C₈-alkoxy, C₁-C₈-alkoxycarbonyl, aryl or heterocyclyl; R² a) is C₁-C₈-alkyl, C₃-C₈-cydoalkyl, C₁-C₈-alkylsulphonyl, C₃-C₈-cydoalkylsulphonyl, aryl-C₀-C₈-alkylsulphonyl, heterocyclylsulphonyl, C₃-C₁₂-cycloalkyl-C₁-C₈-alkanoyl, C₃-C₁₂-cycloalkyl-C₃-C₈-cycloalkanoyl, aryl-C₁-C₈-alkanoyl, heterocydyl-C₁-C₈-alkanoyl, aryl-C₃-C₈-cycloalkanoyl, C₁-C₈-alkanoyl, C₁-C₈-alkoxycarbonyl, optionally N-mono or N,N-di-C₁-C₈-alkylated carbamoyl-C₀-C₈-alkyl, aryl-C₀-C₄-alkyl or heterocyclyl-C₀-C₄-alkyl, which radicals may be substituted by 1-4 C₁-C₈-alkyl, C₃-C₈-cydoalkyl, C₃-C₈-cydoalkoxy, amino, C₁₋₆-alkylamino, di-C₁₋₆-alkylamino, C₀-C₆-alkylcarbonylamino, halogen, cyano, hydroxyl, oxide, oxo, trifluoromethyl, C₁-C₈-alkoxy, optionally N-mono or N,N-di-C₁-C₈-alkylated carbamoyl, C₁-C₈-alkoxycarbonyl, C₁₋₆-alkylenedioxy, aryl or heterocyclyl; or
b) together with R₁ and the nitrogen atom to which they are bonded, is a saturated or partly unsaturated 4-8-membered heterocyclic ring which may contain an additional nitrogen, oxygen or sulphur atom or an -SO- or -SO2- group, and the additional nitrogen atom may optionally be substituted by C₁-C₈-alkyl, C₁-C₈-alkanoyl, C₁-C₈-alkoxycarbonyl, aryl or heterocyclyl radicals, in which case this heterocyclic ring may be part of a bicyclic or tricyclic ring system having a total of up to 16 members and the second ring may also contain a nitrogen, oxygen or sulphur atom or an -SO- or -SO2- group, and the nitrogen atom of the second ring may optionally be substituted by C₁-C₈-alkyl, C₁-C₈-alkanoyl, C₁-C₈-alkoxycarbonyl, aryl or heterocyclyl radicals, and all ring systems mentioned may be substituted by 1-4 C₁-C₈-alkyl, halogen, hydroxyl, oxide, oxo, trifluoromethyl, C₁-C₈-alkoxy, C₁-C₈-alkoxy-C₁-C₈-alkyl, C₁-C₈-alkoxy-C₁-C₈-alkoxy, C₁-C₈-alkoxycarbonylamino, C₁-C₈-alkylcarbonylamino, C₁-C₈-alkylamino, N,N-di-C₁-C₈-alkylamino, aryl-C₀-C₄-alkyl, aryloxy-C₀-C₄-alkyl, aryl-C₀-C₄-alkyl-C₁-C₈-alkoxy, aryloxy-C₀-C₄-alkyl-C₁-C₈-alkoxy, heterocyclyl-C₀-C₄-alkyl, heterocyclyloxy-C₀-C₄-alkyl, heterocyclyl-C₀-C₄-alkyl-C₁-C₈-alkoxy or heterocyclyloxy-C₀-C₄-alkyl-C₁-C₈-alkoxy;
   R³ is hydrogen, C₁-C₄-alkyl, C₁-C₈-alkoxycarbonyl or C₁-C₈-alkanoyl;
   R⁴ is hydrogen, C₁-C₄-alkyl, C₁-C₈-alkoxycarbonyl or C₁-C₈-alkanoyl;
   R⁵ are each independently hydrogen, C₁-C₈-alkyl or, together with the carbon atom to which they are bonded, are a C₃-C₈-cydoalkylidene radical; and
   (A) R⁶ is a heterocyclyl radical or a polycyclic, unsaturated hydrocarbon radical which is substituted by from one to four radicals selected from C₁-C₆-alkyl, C₃₋₈-cydoalkyl, C₃₋₈-cydoalkoxy, C₃₋₈-cycloalkoxy-C₁₋₆-alkyl, C₃₋₈-cydoalkoxy-C₁₋₆-alkoxy, C₁-C₆-alkylamino, di-C₁-C₆-alkylamino, amino-C₁₋₆-alkyl, amino-C₂₋₇-alkoxy, polyhalo-C₁₋₆-alkyl, polyhalo-C₂₋₇-alkoxy, nitro, amino, C₂-C₆-alkenyl, C₁-C₆-alkoxy, C₁-C₆-alkanoyloxy, hydroxyl, halogen, oxide, oxo, cyano, carbamoyl, carboxy, C₁-C₆-alkylenedioxy, phenyl, phenoxy, phenylthio, phenyl-C₁-C₆-alkyl or phenyl-C₁-C₆-alkoxy, each of which are optionally substituted by halogen, C₁-C₆-alkyl, C₁₋₆-alkoxy, hydroxyl, C₁-C₆-alkylamino, di-C₁-C₆-alkylamino, C₁₋₆-alkoxycarbonyl, hydroxy-C₁₋₆-alkyl or trifluoromethyl, pyridylcarbonylamino-C₁₋₆-alkyl, C₂₋₇-alkenyloxy, C₁₋₆-alkoxy-C₁₋₆-alkoxy, C₁₋₆-alkoxy-C₁₋₆-alkoxy-C₁₋₆-alkyl, methoxybenzyloxy, hydroxybenzyloxy, methylenedioxybenzyloxy, dioxolanyl-C₁₋₆-alkoxy, C₃₋₈-cydoalkyl-C₁₋₆-alkyl, C₃₋₈-cycloalkyl-C₁₋₆-alkoxy, hydroxy-C₂₋₇-alkoxy, carbamoyloxy-C₂₋₇-alkoxy, pyridyl-carbamoyloxy-C₂₋₇-alkoxy, benzoyloxy-C₂₋₇-alkoxy, C₁₋₆-alkoxycarbonyl, C₁₋₆alkylcarbonylamino, C₁₋₆-alkylcarbonylamino-C₁₋₆-alkyl, C₁₋₆-alkylcarbonylamino-C₂₋₇-alkoxy, (N-C₁₋₆-alkyl)-C₁₋₆-alkylcarbonylamino-C₁₋₆-alkyl, (N-C₁₋₆-alkyl)-C₁₋₆-alkylcarbonylamino-C₂₋₇-alkoxy, C₃₋₈-cycloalkylcarbonylamino-C₁₋₆-alkyl, C₃₋₈-cycloalkylcarbonylamino-C₂₋₇-alkoxy, C₁₋₆-alkoxy-C₁₋₆-alkyl, hydroxy-C₁₋₆-alkyl, hydroxy-C₂₋₇-alkoxy-C₁₋₆-alkyl, hydroxy-C₂₋₇-alkoxy-C₁₋₆-alkoxy, C₁₋₆-alkoxycarbonylamino-C₁₋₆-alkyl, C₁₋₆-alkoxycarbonylamino-C₂₋₇-alkoxy, C₁₋₆-alkylaminocarbonylamino-C₁₋₆-alkyl, C₁₋₆-alkylaminocarbonylamino-C₂₋₇-alkoxy, C₁₋₆-alkylaminocarbonyl-C₁₋₆-alkyl, C₁₋₆-alkylaminocarbonyl-C₁₋₆-alkoxy, C₁₋₆-alkylaminocarbonyl-C₁₋₆-alkoxy-C₁₋₆-alkyl, di-C₁₋₆alkylaminocarbonyl-C₁₋₆-alkyl, di-C₁₋₆-alkylaminocarbonyl-C₁₋₆-alkoxy, C₁₋₆-alkylcarbonyloxy-C₁₋₆-alkyl, C₁₋₆-alkylcarbonyloxy-C₂₋₆-alkoxy, cyano-C₁₋₆-alkyl, cyano-C₁₋₆-alkoxy, 2-oxooxazolidinyl-C₁₋₆-alkyl, 2-oxooxazolidinyl-C₁₋₆-alkoxy, C₁₋₆-alkoxycarbonyl-C₁₋₆-alkyl, C₁₋₆-alkoxycarbonyl-C₁₋₆-alkoxy, C₁₋₆-alkylsulphonylamino-C₁₋₆-alkyl, C₁₋₆-alkylsulphonylamino-C₂₋₇-alkoxy, (N-C₁₋₆-alkyl)-C₁₋₆-alkylsulphonylamino-C₁₋₆-alkyl, (N-C₁₋₆-alkyl)-C₁₋₆-alkylsulphonylamino-C₂₋₇-alkoxy, C₁₋₆-alkylamino-C₁₋₆-alkyl, C₁₋₆-alkylamino-C₂₋₇-alkoxy, di-C₁₋₆-alkylamino-C₁₋₆-alkyl, di-C₁₋₆-alkylamino-C₂₋₇-alkoxy, C₁₋₆-alkylsulphonyl-C₁₋₆-alkyl, C₁₋₆-alkylsulphonyl-C₁₋₆-alkoxy, carboxy-C₁₋₆-alkyl, carboxy-C₁₋₆-alkoxy, carboxy-C₁₋₆-alkoxy-C₁₋₆-alkyl, C₁₋₆-alkoxy-C₁₋₆-alkylcarbonyl, acyl-C₁₋₆-alkoxy-C₁₋₆-alkyl, (N-C₁₋₆-alkyl)-C₁₋₆-alkoxycarbonylamino, (N-hydroxy)-C₁₋₆-alkylaminocarbonyl-C₁₋₆-alkyl, (N-hydroxy)-C₁₋₆-alkylaminocarbonyl-C₁₋₆-alkoxy, (N-hydroxy)aminocarbonyl-C₁₋₆-alkyl, (N-hydroxy)aminocarbonyl-C₁₋₆-alkoxy, C₁₋₆-alkoxy-aminocarbonyl-C₁₋₆-alkyl, 6-alkoxyaminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆-alkylaminocarbonyl-C₁₋₆-alkoxy, (N-acyl)-C₁₋₆-alkoxy-C₁₋₆-alkylamino, C₁₋₆-alkoxy-C₁₋₆-alkylcarbamoyl, (N-C₁₋₆-alkyl)-C₁₋₆-alkoxy-C₁₋₆-alkylcarbamoyl, C₁₋₆-alkoxy-C₁₋₆-alkylcarbonyl, C₁₋₆-alkoxy-C₁₋₆-alkylcarbonylamino, (N-C₁₋₆-alkyl)-C₁₋₆-alkoxy-C₁₋₆-alkylcarbonylamino, 1-C₁₋₆-alkoxy-C₁₋₆-alkylimidazol-2-yl, 1-C₁₋₆-alkoxy-C₁₋₆-alkyltetrazol-5-yl, 5-C₁₋₆-alkoxy-C₁₋₆-alkyltetrazol-1-yl, 2-C₁₋₆-alkoxy-C₁₋₆-alkyl-4-oxoimidazol-1-yl, carbamoyl-C₁₋₆-alkyl, carbamoyl-C₁₋₆-alkoxy, C₁₋₆-alkylcarbamoyl, di-C₁₋₆-alkylcarbamoyl, C₁₋₆-alkylsulphonyl, C₁₋₆-alkylamidinyl, acetamidinyl-C₁₋₆-alkyl, O-methyloximyl-C₁₋₆-alkyl, O,N-dimethylhydroxylamino-C₁₋₆-alkyl, C₃₋₆-cycloalkyl-C₁₋₆-alkanoyl, aryl-C₁₋₆-alkanoyl or heterocyclyl-C₁₋₆-alkanoyl, or else pyridyl, pyridyloxy, pyridylthio, pyridylamino, pyridyl-C₁₋₆-alkyl, pyridyl-C₁₋₆-alkoxy, pyrimidinyl, pyrimidinyloxy, pyrimidinylthio, pyrimidinylamino, pyrimidinyl-C₁₋₆-alkyl, pyrimidinyl-C₁₋₆-alkoxy, thienyl, thienyl-C₁₋₆-alkyl, thienyl-C₁₋₆-alkoxy, furyl, furyl-C₁₋₆-alkyl or furyl-C₁₋₆-alkoxy, each of which is optionally substituted by halogen, C₁₋₆-alkyl, C₁₋₆-alkoxy or dihydroxy-C₁₋₆-alkylaminocarbonyl, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1 ,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyl-tetrazol-1-ylalkyl, 5-methyl-tetrazol-1-ylalkoxy, thiazol-4-ylalkyl, thiazol-4-ylalkoxy, oxazol-4-ylalkyl, oxazol-4-ylalkoxy, 2-oxo-pyrrolidinylalkyl, 2-oxo-pyrrolidinylalkoxy, imidazolylalkyl, imidazolylalkoxy, 2-methyl-imidazolylalkyl, 2-methyl-imidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₆-alkoxy-C₁₋₆-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl, 2-oxotetrahydropyrimidinyl and the -O-CH₂CH(OH)CH₂NRₓ radical where NRₓ is a mono- or di-C₁₋₆-alkylamino, piperidino, morpholino, piperazino or N-methylpiperazino radical; or
   (B) R⁶ is a polycyclic, unsaturated hydrocarbon radical, phenyl substituted by C₁-C₆-alkylenedioxy, furyl, thienyl, pyridyl, pyrimidyl, indolyl, quinolinyl, pyrazinyl, triazolyl, imidazolyl, benzothiazolyl, pyranyl, tetrahydropyranyl, azetidinyl, morpholinyl, tetrahydroquinolinyl, tetrahydroisoquinolinyl quinazolinyl, quinoxalinyl, isoquinolyl, benzo[b]thienyl, isobenzofuranyl, benzoimidazolyl, 2-oxobenzoimidazolyl, oxazolyl, thiazolyl, pyrrolyl, pyrazolyl, triazinyl, dihydrobenzofuranyl, 2-oxodihydrobenzo[d] [1,3]oxazinyl, 4-oxodihydroimidazolyl, 5-oxo-4H[1,2,4]triazinyl, 3-oxo-4H-benzo [1,4]thiazinyl, tetrahydroquinoxalinyl, 1,1,3-trioxodihydro-2H-1λ⁶-benzo [1,4]thiazinyl, 1-oxopyridyl, dihydro-3H-benzo[1,4]oxazinyl, 3,4-dihydro-2H-benzo [1,4]oxazinyl, 2-oxotetrahydrobenzo[e][1,4]diazepinyl, 2-oxodihydrobenzo[e] [1,4]diazepinyl, 1H-pyrrolizinyl, phthalazinyl, 1-oxo-3H-isobenzofuranyl, 4-oxo-3H-thieno[2,3-d] pyrimidinyl, 3-oxo-4H-benzo[1,4]oxazinyl, [1,5]naphthyridyl, dihydro-2H-benzo [1,4]thiazinyl, 1,1-dioxodihydro-2H-benzo[1,4]thiazinyl, 2-oxo-1H-pyrido [2,3b] [1,4]oxazinyl, dihydro-1H-pyrido[2,3-b][1,4]oxazinyl, 1H-pyrrolo[2,3-b]pyridyl, benzo[1,3]dioxolyl, benzoxazolyl, 2-oxobenzooxazolyl, 2-oxo-1,3-dihydroindolyl, 2,3-dihydroindolyl, indazolyl, benzofuranyl, dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxopiperidinyl, 2-oxopyrrolidinyl, 2-oxo[1,3]oxazinyl, 2-oxoazepanyl, or 2-oxotetrahydropyrimidinyl Aryl, and aryl in aryl-C₀-C₄-alkyl, aryloxy-C₀-C₄-alkyl, aryl-C₀-C₄-alkyl-C₁-C₈-alkoxy and aryloxy-C₀-C₄-alkyl-C₁-C₈-alkoxy, contains generally 1-14, preferably 6-10, carbon atoms, and is, for example, phenyl, indenyl, e.g. 2- or 4-indenyl, or naphthyl, e.g. 1- or 2-naphthyl. Preference is given to aryl having 6-10 carbon atoms, in particular phenyl or 1- or 2-naphthyl. The radicals mentioned may be unsubstituted or, for example, mono- or polysubstituted, for example mono- or disubstituted, by C₁-C₈-alkyl, cyano, halogen, oxo, trifluoromethyl, C₁-C₈-alkoxy, C₁-C₈-alkoxy-C₁-C₈-alkoxy, C₁-C₈-alkoxy-C₁-C₈-alkyl, C₁-C₈-alkoxycarbonyl, aryl or heteroaryl, and the substituent may be in any position, for example in the o-, m- or p-position of the phenyl radical, or in the 3- or 4-position of the 1- or 2-naphthyl radical, and a plurality of identical or different substituents may also be present.

Aryl-C₀-C₄-alkyl is, for example, phenyl, naphthyl or benzyl.

Examples of substituents on R⁶ in the definition of heterocyclyl radicals and polycyclic, unsaturated hydrocarbon radicals are C₁-C₆-alkyl, C₃₋₈-cycloalkyl, C₃₋₈-cycloalkoxy, C₃₋₈-cycloalkoxy-C₁₋₆-alkyl, C₃₋₈-cycloalkoxy-C₁₋₆-alkoxy, C₁-C₆-alkylamino, di-C₁-C₆-alkylamino, amino-C₁₋₆-alkyl, amino-C₂₋₇-alkoxy, polyhalo-C₁₋₆-alkyl, especially trifluoromethyl, polyhalo-C₂₋₇-alkoxy, nitro, amino, C₂-C₆-alkenyl, C₁-C₆-alkoxy, C₁-C₆-alkanoyloxy, hydroxyl, halogen, oxide, oxo, cyano, carbamoyl, carboxy, C₁-C₆-alkylenedioxy, phenyl, phenoxy, phenylthio, phenyl-C₁-C₆-alkyl or phenyl-C₁-C₆-alkoxy, each of which is optionally substituted by halogen, C₁-C₆-alkyl, C₁₋₆-alkoxy, hydroxyl, C₁-C₆-alkylamino, di-C₁-C₆-alkylamino, C₁₋₆-alkoxycarbonyl, hydroxy-C₁₋₆-alkyl or trifluoromethyl, C₁₋₆-alkoxycarbonylphenyl, hydroxy-C₁₋₆-alkylphenyl, benzyloxy, pyridylcarbonylamino-C₁₋₆-alkyl, C₂₋₆-alkenyloxy, C₁₋₆-alkoxy-C₁₋₆-alkoxy, C₁₋₆-alkoxy-C₁₋₆-alkoxy-C₁₋₆-alkyl, methoxybenyloxy, hydroxybenzyloxy, phenethyloxy, methylenedioxybenzyloxy, dioxolanyl-C₁₋₆-alkoxy, cyclopropyl-C₁₋₆-alkyl, cyclopropyl-C₁₋₆-alkoxy, hydroxy-C₂₋₇-alkoxy, carbamoyloxy-C₁₋₆-alkoxy, pyridylcarbamoyloxy-C₁₋₆-alkoxy, benzoyloxy-C₁₋₆-alkoxy, C₁₋₆-alkoxycarbonyl, C₁₋₆-alkylcarbonylamino, C₁₋₆-alkylcarbonylamino-C₁₋₆-alkyl, C₁₋₆-alkylcarbonylamino-C₂₋₇-alkoxy, (N-C₁₋₆-alkyl)-C₁₋₆-alkylcarbonylamino-C₁₋₆-alkyl, (N-C₁₋₆-alkyl)-C₁₋₆-alkylcarbonylamino-C₂₋₇-alkoxy, C₃₋₈-cycloalkylcarbonylamino-C₁₋₆-alkyl, C₃₋₈-cycloalkylcarbonylamino-C₂₋₇-alkoxy, C₁₋₆-alkoxy-C₁₋₆-alkyl, hydroxy-C₁₋₆-alkyl, hydroxy-C₂₋₇-alkoxy-C₁₋₆-alkyl, hydroxy-C₂₋₇-alkoxy-C₁₋₆-alkoxy, C₁₋₆-alkoxycarbonylamino-C₁₋₆-alkyl, C₁₋₆-alkoxycarbonylamino-C₂₋₇-alkoxy, C₁₋₆-alkylaminocarbonylamino-C₁₋₆-alkyl, C₁₋₆-alkylaminocarbonylamino-C₂₋₇-alkoxy, C₁₋₆-alkylaminocarbonyl-C₁₋₆-alkyl, C₁₋₆-alkylaminocarbonyl-C₁₋₆-alkoxy, C₁₋₆-alkylaminocarbonyl-C₁₋₆-alkoxy-C₁₋₆-alkyl, di-C₁₋₆-alkylaminocarbonyl-C₁₋₆-alkyl, di-C₁₋₆-alkylaminocarbonyl-C₁₋₆-alkoxy, C₁₋₆-alkylcarbonyloxy-C₁₋₆-alkyl, C₁₋₆-alkylcarbonyloxy-C₁₋₆-alkoxy, cyano-C₁₋₆-alkyl , cyano-C₁₋₆-alkoxy, 2-oxooxazolidinyl-C₁₋₆-alkyl, 2-oxooxazolidinyl-C₁₋₆-alkoxy, C₁₋₆-alkoxycarbonyl-C₁₋₆-alkyl, C₁₋₆-alkoxycarbonyl-C₁₋₆-alkoxy, C₁₋₆-alkylsulphonylamino-C₁₋₆-alkyl, C₁₋₆-alkylsulphonylamino-C₂₋₇-alkoxy, (N-C₁₋₆-alkyl)-C₁₋₆-alkylsulphonylamino-C₁₋₆-alkyl, (N-C₁₋₆-alkyl)-C₁₋₆-alkylsulphonylamino-C₂₋₇-alkoxy, C₁₋₆-alkylamino-C₁₋₆-alkyl, C₁₋₆-alkylamino-C₂₋₇-alkoxy, di-C₁₋₆-alkylamino-C₁₋₆-alkyl, di-C₁₋₆-alkylamino-C₂₋₇-alkoxy, C₁₋₆-alkylsulphonyl-C₁₋₆-alkyl, C₁₋₆-alkylsulphonyl-C₁₋₆-alkoxy, carboxy-C₁₋₆-alkyl, carboxy-C₁₋₆-alkoxy, carboxy-C₁₋₆-alkoxy-C₁₋₆-alkyl, C₁₋₆-alkoxy-C₁₋₆-alkylcarbonyl, acyl-C₁₋₆-alkoxy-C₁₋₆-alkyl, (N-C₁₋₆-alkyl)-C₁₋₆-alkoxycarbonylamino, (N-hydroxy)-C₁₋₆-alkylaminocarbonyl-C₁₋₆-alkyl, (N-hydroxy)-C₁₋₆-alkylaminocarbonyl-C₁₋₆-alkoxy, (N-hydroxy)aminocarbonyl-C₁₋₆-alkyl, (N-hydroxy)aminocarbonyl-C₁₋₆-alkoxy, C₁₋₆-alkoxyaminocarbonyl-C₁₋₆-alkyl, 6-alkoxy-aminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆-alkylaminocarbonyl-C₁₋₆-alkoxy, (N-acyl)-C₁₋₆-alkoxy-C₁₋₆-alkylamino, C₁₋₆-alkoxy-C₁₋₆-alkylcarbamoyl, (N-C₁₋₆-alkyl)-C₁₋₆-alkoxy-C₁₋₆-alkylcarbamoyl, C₁₋₆-alkoxy-C₁₋₆-alkylcarbonyl, C₁₋₆-alkoxy-C₁₋₆-alkylcarbonylamino, (N-C₁₋₆-alkyl)-C₁₋₆-alkoxy-C₁₋₆-alkylcarbonylamino, 1-C₁₋₆-alkoxy-C₁₋₆-alkylimidazol-2-yl, 1-C₁₋₆-alkoxy-C₁₋₆-alkyltetrazol-5-yl, 5-C₁₋₆-alkoxy-C₁₋₆-alkyltetrazol-1-yl, 2-C₁₋₆-alkoxy-C₁₋₆-alkyl-4-oxoimidazol-1-yl, carbamoyl-C₁₋₆-alkyl, carbamoyl-C₁₋₆-alkoxy, C₁₋₆-alkylcarbamoyl, di-C₁₋₆-alkylcarbamoyl, C₁₋₆-alkylsulphonyl, C₁₋₆-alkylamidinyl, acetamidinyl-C₁₋₆-alkyl, O-methyloximyl-C₁₋₆-alkyl, O,N-dimethylhydroxylamino-C₁₋₆-alkyl, C₃₋₆-cydoalkyl-C₁₋₆-alkanoyl, aryl-C₁₋₆-alkanoyl or heterocyclyl-C₁₋₆-alkanoyl; or else pyridyl, pyridyloxy, pyridylthio, pyridylamino, pyridyl-C₁₋₆-alkyl, pyridyl-C₁₋₆-alkoxy, pyrimidinyl, pyrimidinyloxy, pyrimidinylthio, pyrimidinylamino, pyrimidinyl-C₁₋₆-alkyl, pyrimidinyl-C₁₋₆-alkoxy, thienyl, thienyl-C₁₋₆-alkyl, thienyl-C₁₋₆-alkoxy, furyl, furyl-C₁₋₆-alkyl or furyl-C₁₋₆-alkoxy, each of which is optionally substituted by halogen, C₁₋₆-alkyl, C₁₋₆-alkoxy or dihydroxy-C₁₋₆-alkylaminocarbonyl.

The term polycyclic, unsaturated hydrocarbon radical comprises also aromatic radicals and denotes radicals such as naphthyl, cyclohexenophenyl, indanyl and acenaphthyl, for example.

The term heterocyclyl denotes mono- or bicyclic, saturated and unsaturated heterocyclic radicals which have from 1 to 4 nitrogen and/or 1 or 2 sulphur or oxygen atoms and may be mono- or polysubstituted, especially mono-, di- or trisubstituted. In addition, the term encompasses the above oxo-substituted radicals. Examples of heterocyclyl radicals are phenyl substituted by C₁-C₆-alkylenedioxy, pyridyl, thienyl, pyrazinyl, triazolyl, imidazolyl, benzothiazolyl, furyl, pyranyl, pyrimidinyl, quinazolinyl, quinolyl, quinoxalinyl, isoquinolyl, benzo[b]thienyl, isobenzofuranyl, benzoimidazolyl, 2-oxobenzoimidazolyl, oxazolyl, thiazolyl, indolyl, pyrrolyl, pyrazolyl, triazinyl, dihydrobenzofuranyl, 2-oxodihydrobenzo[d][1,3]oxazinyl, 4-oxodihydroimidazolyl, 5-oxo-4H-[1,2,4]triazinyl, 3-oxo-4H-benzo[1,4]thiazinyl, tetrahydroquinoxalinyl, tetrahydroquinolinyl, tetrahydroisoquinolinyl 1,1,3-trioxodihydro-2H-1λ⁶-benzo[1,4]thiazinyl, 1-oxopyridyl, dihydro-3H-benzo[1,4]oxazinyl, 2-oxotetrahydrobenzo [e][1,4]diazepinyl, 2-oxodihydrobenzo[e][1,4]diazepinyl, 1H-pyrrolizinyl, phthalazinyl, 1-oxo-3H-isobenzofuranyl, 4-oxo-3H-thieno[2,3-d]pyrimidinyl, 3,4-dihydro-2H-benzo[1,4]oxazinyl, 3-oxo-4H-benzo[1,4]oxazinyl, [1,5]naphthyridyl, dihydro-2H-benzo[1,4]thiazinyl, 1,1-dioxodihydro-2H-benzo[1,4]thiazinyl, 2-oxo-1H-pyrido[2,3-b][1,4]oxazinyl, dihydro-1H-pyrido [2,3-b][1,4]oxazinyl, 1H-pyrrolo[2,3-b]pyridyl, benzo[1,3]dioxolyl, benzooxazolyl, 2-oxobenzooxazolyl, 2-oxo-1,3-dihydroindolyl, 2,3-dihydroindolyl, indazolyl, pyrrolo [3,2-c]pyridinyl, pyrrolo[2,3-c]pyridinyl, pyrrolo[3,2-b]pyridinyl, [1,2,3]triazolo[1,5-a]pyridinyl, [1,2,4]triazolo[4,3-a]pyridinyl, imidazo[1,2-a]pyrimidinyl,_imimidazo[1,5-a]pyridinyl or benzofuranyl. Examples of saturated heterocyclyl radicals are azepanyl, azetidinyl, aziridinyl, dioxolanyl, dioxanyl, dithiolanyl, dithianyl, oxepanyl, pyrrolidinyl, 1-methylpiperidinyl, 1-methylpyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydrofuranyl, tetrahydropyranyl, tetrahydrothiophenyl, tetrahydrothiopyranyl, 2-oxoimidazolidinyl, 2-oxooxazolidinyl, 2-oxopiperidinyl, 2-oxopyrrolidinyl, 2-oxo[1,3]oxazinyl, 2-oxoazepanyl or 2-oxotetrahydropyrimidinyl.

In the case of R⁶, the heterocyclyl radicals may additionally also be substituted by heterocyclylalkyl, heterocyclylalkoxy, heterocyclylalkoxyalkyl or heterocyclyl, for example piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl[1,2,4]oxadiazol-5-ylalkyl, 3-methyl[1,2,4]oxadiazol-5-ylalkoxy, 5-methyl[1,2,4]oxadiazol-3-ylalkyl, 5-methyl[1,2,4]oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methyl-imidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₆-alkoxy-C₁₋₆-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl or by the -O-CH₂CH(OH)CH₂NRₓ radical where NRₓ is a mono- or di-C₁₋₆-alkylamino, piperidino, morpholino, piperazino or N-methylpiperazino radical.

The term polyhydroxyalkyl denotes C₁-C₇-alkyl radicals which may be substituted by 2-6 hydroxyl groups, for example glyceryl, arabityl, sorbityl, etc.

Heterocyclyl and heterocyclyloxy in heterocyclyl-C₀-C₄-alkyl, heterocyclyloxy-C₀-C₄-alkyl, heterocyclyl-C₀-C₄-alkyl-C₁-C₈-alkoxy and heterocyclyloxy-C₀-C₄-alkyl-C₁-C₈-alkoxy having from 5 to 7 ring atoms in the heterocyclyl ring which contains one ring nitrogen atom and may contain one further ring heteroatom selected from oxygen, sulphur or nitrogen, is, for example, pyridinyl or imidazolyl which are each unsubstituted or substituted by C₁-C₈-alkyl, C₁₋₆-alkoxy-C₁₋₆-alkyl, C₁₋₆-alkoxy-C₁₋₆-alkoxy, halogen, cyano, oxide, oxo, trifluoromethyl, C₁-C₈-alkoxy, C₁-C₈-alkoxycarbonyl, aryl or heterocyclyl.

Heterocyclyl-C₀-C₄-alkyl is, for example, pyridinyl, methylenepyridinyl or imidazolyl.

Heterocyclyl which is bonded via a ring nitrogen atom and has from 4 to 8 ring atoms has in particular from 5 to 7 ring atoms and may have 1 or 2 fused-on phenyl or cycloalkyl radicals, or else be present as the spiro compound. Examples include pyrrolidino, piperidino, morpholino, 9-azabicyclo[3.3.1]non-9-yl, 1-azepan-1-yl, 2,8-diazaspiro[4.5]dec-8-yl, octahydroisoindol-2-yl, 4-azatricyclo[5.2.1.0^{2,6}]dec-4-yl, 3-azabicyclo[3.2.1]oct-3-yl, 3,7-diazabicyclo[3.3.1]non-3-yl, 3-azabicyclo[3.3.1]non-3-yl, 8-azabicyclo[3.2.1]oct-8-yl, 3-azabicyclo[3.2.2]non-3-yl and tetrahydro-1H-1-benz[6,7-b]azepin-1-yl.

In the case of nitrogen heterocycles, the heterocyclyl radicals may be bonded via the nitrogen or via a ring carbon.

Halogen is, for example, fluorine, chlorine, bromine or iodine, preferably fluorine or chlorine.

Polyhalo-C₁-C₄-alkyl is, for example, di-, tri- or tetrahalo-C₁-C₄-alkyl, such as trifluoromethyl.
C₃-C₈-Cycloalkanoyl is preferably 3-, 5- or 6-membered cycloalkyl-C(=O)-.

3- to 8-membered cycloalkoxy is preferably 3-, 5- or 6-membered cycloalkoxy, such as cyclopropyloxy cyclopentyloxy cyclohexyloxy.

3- to 8-membered cycloalkyl is preferably 3-, 5- or 6-membered cycloalkyl, such as cyclopropyl, cyclopentyl, cyclohexyl.

Amino-C₂-C₄-alkoxy is, for example, 2-aminoethoxy, and also 3-aminopropyloxy or 4-aminobutyloxy.

Amino-C₁-C₄-alkyl is, for example, 2-aminoethyl, 3-aminopropyl or 4-aminobutyl.

Carbamoyl-C₀-C₈-alkyl is, for example, carbamoyl, carbamoylmethyl, 2-carbamoylethyl, 3-carbamoylpropyl, 2-(3-carbamoyl)propyl, 2-carbamoylpropyl, 3-(1-carbamoyl)propyl, 2-(2-carbamoyl)propyl, 2-carbamoyl-2-methylpropyl, 4-carbamoylbutyl, 1-carbamoylbutyl, 1-(1-carbamoyl-2-methyl)butyl, 3-(4-carbamoyl-2-methyl)butyl.

Carboxy-C₁-C₄-alkoxy is, for example, carboxymethoxy, 2-carboxyethoxy, 2- or 3-carboxypropyloxy or 4-carboxybutyloxy, especially carboxymethoxy.

Carboxy-C₁-C₄-alkyl is, for example, carboxymethyl, 2-carboxyethyl, 2- or 3-carboxypropyl, 2-carboxy-2-methylpropyl, 2-carboxy-2-ethylbutyl or 4-carboxybutyl, especially carboxymethyl.

Cyano-C₁-C₄-alkoxy is, for example, cyanomethoxy, 2-cyanoethoxy 2- or 3-cyanopropyloxy or 4-cyanobutyloxy, especially cyanomethoxy

Cyano-C₁-C₄-alkyl is, for example, cyanomethyl, 2-cyanoethyl, 2- or 3-cyanopropyl, 2-cyano-2-methylpropyl, 2-cyano-2-ethylbutyl or 4-cyanobutyl, especially cyanomethyl.

N,N-Di-C₁-C₄-alkylamino is, for example, dimethylamino, N-methyl-N-ethylamino, diethylamino, N-methyl-N-propylamino or N-butyl-N-methylamino.

N,N-Di-C₁-C₄-alkylamino-C₂-C₄-alkoxy is 2-dimethylaminoethoxy, 3-dimethylaminopropyloxy, 4-dimethylaminobutyloxy, 2-diethylaminoethoxy, 2-(N-methyl-N-ethylamino)ethoxy or 2-(N-butyl-N-methylamino)ethoxy.

N,N-Di-C₁-C₄-alkylamino-C₁-C₄-alkyl is, for example, 2-dimethylaminoethyl, 3-dimethylaminopropyl, 4-dimethylaminobutyl, 2-diethylaminoethyl, 2-(N-methyl-N-ethylamino)ethyl or 2-(N-butyl-N-methylamino)ethyl.

N,N-Di-C₁-C₄-alkylcarbamoyl-C₁-C₄-alkoxy is, for example, methyl or dimethylcarbamoyl-C₁-C₄-alkoxy, such as N-methyl-, N-butyl- or N,N-dimethylcarbamoylmethoxy, 2-(N-methylcarbamoyl)ethoxy, 2-(N-butylcarbamoyl)ethoxy, 2-(N,N-dimethylcarbamoyl)ethoxy, 3-(N-methylcarbamoyl)propyloxy, 3-(N-butylcarbamoyl) propyloxy, 3-(N,N-dimethylcarbamoyl)propyloxy or 4-(N-methylcarbamoyl)butyloxy, 4-(N-butylcarbamoyl)butyloxy or 4-(N,N-dimethylcarbamoyl)butyloxy, especially N-methyl-, N-butyl- or N,N-dimethylcarbamoylmethoxy.

N,N-Di-C₁-C₄-alkylcarbamoyl-C₁-C₄-alkyl is, for example, 2-dimethylcarbamoylethyl, 3-dimethylcarbamoylpropyl, 2-dimethylcarbamoylpropyl, 2-(dimethylcarbamoyl)-2-methylpropyl or 2-(1-dimethylcarbamoyl)-3-methylbutyl.

Optionally partially hydrogenated pyridyl- or N-oxidopyridyl-C₁-C₄-alkoxy is, for example, pyridyl- or N-oxidopyridylmethoxy, 2-pyridylethoxy 2- or 3-pyridylpropyloxy or 4-pyridylbutyloxy, in particular 3- or 4-pyridylmethoxy.

Optionally partially hydrogenated pyridyl- or N-oxidopyridyl-C₁-C₄-alkyl is, for example, pyridyl- or N-oxidopyridylmethyl, 2-pyridylethyl, 2- or 3-pyridylpropyl or 4-pyridylbutyl, in particular 3- or 4-pyridylmethyl.

Halo-C₂-C₈-(hydroxy)alkoxy is, for example, halo-C₂-C₄-(hydroxy)alkoxy, such as 3-halo-, such as 3-chloro-2-hydroxypropyloxy.

Hydroxy-C₂-C₈-alkoxy is, for example, hydroxy-C₂-C₄-alkoxy such as 2-hydroxybutyloxy, 3-hydroxypropyloxy or 4-hydroxybutyloxy.

Hydroxy-C₂-C₈-alkyl is, for example, hydroxy-C₂-C₄-alkyl, such as 2-hydroxyethyl, 3-hydroxypropyl or 4-hydroxybutyl.

Morpholino-C₁-C₄-alkoxy may be N-oxidized and is, for example, 1-morpholinoethoxy, 3-morpholinopropyloxy or 1-(morpholino-2-methyl)propyloxy.

Morpholino-C₁-C₄-alkyl may be N-oxidized and is, for example, morpholinomethyl, 2-morpholinoethyl, 3-morpholinopropyl or 1- or 2-(4-morpholino)butyl.

C₁-C₈-Alkanoyl is in particular C₂-C₆-alkanoyl, such as acetyl, propionyl, butyryl, isobutyryl or pivaloyl.

N-C₁-C₄-Alkanoylamino-C₁-C₄-alkyl is, for example, N-C₁-C₄-alkanoylamino-C₁-C₄-alkyl, such as 2-acetaminoethyl.

C₁-C₈-Alkanoyl-C₂-C₄-alkoxy (oxo-C₂-C₈-alkoxy) bears the C₁-C₈-alkanoyl group in a position higher than the α-position and is, for example, 4-acetylbutoxy.

C₁-C₈-Alkanoyloxy-C₁-C₄-alkyl bears the C₁-C₈-alkanoyloxy group in a position higher than the α-position and is, for example, 4-acetoxybutyl.

C₁-C₈-Alkanesulphonyl-C₁-C₄-(hydroxy)alkoxy is, for example, 3-methanesulphonyl-2-hydroxypropyloxy.

C₁-C₈-Alkanesulphonyl-C₁-C₄-alkoxy is, for example, methanesulphonylmethoxy or 3-methanesulphonyl-2-hydroxypropyloxy.

C₁-C₈-Alkanesulphonylamino-C₂-C₄-alkoxy is, for example, 2-ethanesulphonylaminoethoxy, 3-ethanesulphonylaminopropyloxy or 3-(1,1-dimethylethanesulphonylamino)propyloxy.

C₁-C₄-Alkanesulphonylamino-C₁-C₄-alkyl is, for example, ethanesulphonylaminomethyl, 2-ethanesulphonylaminoethyl, 3-ethanesulphonylaminopropyl or 3-(1,1-dimethylethanesulphonylamino)propyl.

C₁-C₈-Alkanesulphonyl-C₁-C₄-alkyl is, for example, ethanesulphonylmethyl, 2-ethanesulphonylethyl, 3-ethanesulphonylpropyl or 3-(1,1-dimethylethanesulphonyl)propyl.

C₂-C₈-alkenyloxy is, for example, allyloxy.

C₂-C₈-Alkenyloxy-C₁-C₄-alkoxy is, for example, allyloxymethoxy.

C₂-C₈-Alkenyloxy-C₁-C₄-alkyl is, for example, allyloxymethyl.

C₁-C₈-Alkoxy is, for example, C₁-C₅-alkoxy such as methoxy, ethoxy, propyloxy, isopropyloxy, butyloxy, isobutyloxy, sec-butyloxy, tert-butyloxy or pentyloxy, but may also be a hexyloxy or heptyloxy group.

C₁-C₈-Alkoxycarbonyl is preferably C₂-C₅-alkoxycarbonyl such as methoxycarbonyl, ethoxycarbonyl, propyloxycarbonyl, isopropyloxycarbonyl, butyloxycarbonyl, isobutyloxycarbonyl, sec-butyloxycarbonyl or tert-butyloxycarbonyl.

C₁-C₈-Alkoxycarbonylamino-C₂-C₈-alkoxy is preferably C₂-C₅-alkoxycarbonylamino-C₂-C₈-alkoxy such as methoxycarbonylamino-C₂-C₈-alkoxy, ethoxycarbonylamino-C₂-C₈-alkoxy, propyloxycarbonylamino-C₂-C₈-alkoxy, isopropyloxycarbonylamino-C₂-C₈-alkoxy, butyloxycarbonylamino-C₂-C₈-alkoxy, isobutyloxycarbonylamino-C₂-C₈-alkoxy, sec-butyloxycarbonylamino-C₂-C₈-alkoxy or tert-butyloxycarbonylamino-C₂-C₈-alkoxy in which C₂-C₈-alkoxy is, for example, ethoxy, propyloxy, butyloxy, pentyloxy or hexyloxy.

C₁-C₈-Alkoxycarbonylamino-C₂-C₈-alkyl is preferably C₂-C₅-alkoxycarbonylamino-C₂-C₈-alkyl such as methoxycarbonylamino-C₂-C₈-alkyl, ethoxycarbonylamino-C₂-C₈-alkyl, propyloxycarbonylamino-C₂-C₈-alkyl, isopropyloxycarbonylamino-C₂-C₈-alkyl, butyloxycarbonylamino-C₂-C₈-alkyl, isobutyloxycarbonylamino-C₂-C₈-alkyl, sec-butyloxycarbonylamino-C₂-C₈-alkyl or tert-butyloxycarbonylamino-C₂-C₈-alkyl, in which C₂-C₈-alkyl is, for example, ethyl, propyl, butyl, pentyl or hexyl.

C₁-C₄-Alkoxycarbonyl-C₁-C₄-alkoxy is, for example, methoxycarbonyl- or ethoxycarbonylmethoxy, 2-methoxycarbonyl- or 2-ethoxycarbonylethoxy, 2- or 3-methoxycarbonyl- or 2- or 3-ethoxycarbonylpropyloxy or 4-methoxycarbonyl- or 4-ethoxycarbonylbutyloxy, in particular methoxycarbonyl- or ethoxycarbonylmethoxy or 3-methoxycarbonyl- or 3-ethoxycarbonylpropyloxy.

C₁-C₄-Alkoxycarbonyl-C₁-C₄-alkyl is, for example, methoxycarbonyl- or ethoxycarbonylmethyl, 2-methoxycarbonyl- or 2-ethoxycarbonylethyl, 3-methoxycarbonyl- or 3-ethoxycarbonylpropyl or 4-ethoxycarbonylbutyl.

C₁-C₄-Alkoxy-C₂-C₄-alkenyl is, for example, 4-methoxybut-2-enyl.

C₁-C₈-Alkoxy-C₁-C₈-alkoxy is, for example, 2-methoxy-, 2-ethoxy- or 2-propyloxyethoxy, 3-methoxy- or 3-ethoxypropyloxy or 4-methoxybutyloxy, in particular 3-methoxypropyloxy or 4-methoxybutyloxy.

C₁-C₆-Alkoxy-C₁-C₆-alkoxy-C₁-C₆-alkyl is, for example, C₁-C₄-alkoxy-C₁-C₄-alkoxy-C₁-C₄-alkyl, such as 2-methoxy-, 2-ethoxy- or 2-propyloxyethoxymethyl, 2-(2-methoxy-, 2-ethoxy- or 2-propyloxyethoxy)ethyl, 3-(3-methoxy- or 3-ethoxypropyloxy)propyl or 4-(2-methoxybutyloxy)butyl, in particular 2-(3-methoxypropyloxy)ethyl or 2-(4-methoxybutyloxy)ethyl.

C₁-C₄-Alkoxy-C₁-C₄-alkyl is, for example, ethoxymethyl, propyloxymethyl, butyloxymethyl, 2-methoxy-, 2-ethoxy- or 2-propyloxyethyl, 3-methoxy- or 3-ethoxypropyl or 4-methoxybutyl, in particular 3-methoxypropyl or 4-methoxybutyl.

C₁-C₈-Alkyl may be straight-chain or branched and is, for example, methyl, ethyl, propyl, isopropyl, butyl, isobutyl, sec-butyl, tert-butyl, or a pentyl, hexyl or heptyl group.

C₁-C₄-Alkylamino-C₂-C₄-alkoxy is, for example, 2-methylamino-, 2-ethylamino-, 2-propylamino- or 2-butylaminoethoxy, 3-ethylamino- or 3-propylaminopropyloxy or 4-methylaminobutoxy.

C₁-C₄-Alkylamino-C₁-C₄-alkyl is, for example, propylaminomethyl, 2-methylamino-, 2-ethylamino-, 2-propylamino- or 2-butylaminoethyl, 3-ethylamino- or 3-propylaminopropyl, or 4-methylaminobutyl.

N-C₁-C₈-Alkylcarbamoyl-C₁-C₄-alkoxy is, for example, methyl- or dimethylcarbamoyl-C₁-C₄-alkoxy, for example methylcarbamoylmethoxy, 2-methylcarbamoylethoxy or 3-methylcarbamoylpropyloxy.

C₁-C₄-Alkylenedioxy is, for example, methylenedioxy or ethylenedioxy, but may also be 1,3- or 1,2-propylenedioxy.

C₁-C₄-Alkylthio-C₁-C₄-(hydroxy)alkoxy is, for example, 2-hydroxy-3-methylthiopropyloxy.

C₁-C₄-Alkylthio-C₁-C₄-alkoxy is, for example, methylthio-C₁-C₄-alkoxy, e.g. methylthiomethoxy, 2-methylthioethoxy or 3-methylthiopropyloxy.

C₁-C₄-Alkylthio-C₁-C₄-alkyl is, for example, methylthio-C₁-C₄-alkyl, e.g. methylthiomethyl, 2-methylthioethyl or 3-methylthiopropyl.

N'-C₂-C₈-Alkanoylpiperazino-C₁-C₄-alkyl is, for example, 4-acetylpiperazinomethyl.

N'-C₁-C₄-Alkylpiperazino-C₁-C₄-alkyl is 4-methylpiperazinomethyl.

Piperazino-C₁-C₄-alkyl is, for example, piperazinomethyl, 2-piperazinoethyl or 3-piperazinopropyl.

Piperidino-C₁-C₄-alkoxy is, for example, piperidinomethoxy, 2-piperidinoethoxy or 3-piperidinopropyloxy

Piperidino-C₁-C₄-alkyl is, for example, piperidinomethyl, 2-piperidinoethyl or 3-piperidinopropyl.

Pyrrolidino-C₂-C₄-alkoxy is, for example, 2-pyrrolidinoethoxy or 3-pyrrolidinopropyloxy.

Pyrrolidino-C₁-C₄-alkyl is, for example, pyrrolidino-C₁-C₄-alkyl, such as pyrrolidinomethyl, 2-pyrrolidinoethyl or 3-pyrrolidinopropyl.

S,S-Dioxothiomorpholino-C₁-C₄-alkyl is, for example, S,S-dioxothiomorpholinomethyl or 2-(S,S-dioxo)thiomorpholinoethyl.

S-Oxothiomorpholino-C₁-C₄-alkyl is, for example, S-oxothiomorpholinomethyl or 2-(S-oxo)thiomorpholinoethyl.

Thiazolyl-C₁-C₄-alkoxy is, for example, thiazolylmethoxy, 2-thiazolylethoxy or 3-thiazolylpropyloxy.

Thiomorpholino-C₁-C₄-alkyl or S,S-dioxothiomorpholino-C₁-C₄-alkyl is, for example, thiomorpholino-C₁-C₄-alkyl such as -methyl or -ethyl, or S,S-dioxothiomorpholino-C₁-C₄-alkyl, such as -methyl or -ethyl.

Salts of compounds having salt-forming groups are in particular acid addition salts, salts with bases or, in the presence of a plurality of salt-forming groups, in some cases also mixed salts or internal salts.

Salts are primarily the pharmaceutically usable or nontoxic salts of compounds of the formula (I).

Such salts are formed, for example, from compounds of the formula (I) with an acidic group, for example a carboxyl or sulpho group, and are, for example, the salts thereof with suitable bases, such as nontoxic metal salts derived from metals of group Ia, Ib, IIa and IIb of the Periodic Table of the Elements, for example alkali metal, in particular lithium, sodium or potassium salts, alkaline earth metal salts, for example magnesium or calcium salts, and also zinc salts or ammonium salts, including those salts which are formed with organic amines, such as optionally hydroxy-substituted mono-, di- or trialkylamines, in particular mono-, di- or tri(lower alkyl)amines, or with quaternary ammonium bases, for example methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amines, such as N,N-dimethyl-N-(2-hydroxyethyl)amine, or N-methyl-D-glucamine, or quaternary ammonium hydroxides, such as tetrabutylammonium hydroxide. The compounds of the formula (I) having a basic group, for example an amino group, may form acid addition salts, for example with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic, sulpho or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the α-amino acids mentioned above, and also methanesulphonic acid, ethanesulphonic acid, 2-hydroxyethanesulphonic acid, ethane-1,2-disulphonic acid, benzenesulphonic acid, 4-toluenesulphonic acid, naphthalene-2-sulphonic acid, 2- or 3-phosphoglycerate, glucose-6-phosphate, N-cyclohexylsulphamic acid (with formation of cyclamates) or with other acidic organic compounds such as ascorbic acid. Compounds of the formula (I) with acidic and basic groups may also form internal salts.

For the isolation and purification, pharmaceutically unsuitable salts may also find use.

Prodrug derivatives of the compounds described in the present context are derivatives thereof which, on *in vivo* application, release the original compound by a chemical or physiological process. A prodrug may be converted to the original compound, for example, when a physiological pH is attained or by enzymatic conversion. Prodrug derivatives may, for example, be esters of freely available carboxylic acids, S- and O-acyl derivatives of thiols, alcohols or phenols, and the acyl group is as defined in the present context. Preference is given to pharmaceutically usable ester derivatives which are converted by solvolysis in physiological medium to the original carboxylic acid, for example lower alkyl esters, cycloalkyl esters, lower alkenyl esters, benzyl esters, mono- or disubstituted lower alkyl esters such as lower ω-(amino, mono- or dialkylamino, carboxyl, lower alkoxycarbonyl)-alkyl esters or such as lower α-(alkanoyloxy, alkoxycarbonyl or dialkylaminocarbonyl)-alkyl esters; as such, pivaloyloxymethyl esters and similar esters are utilized in a conventional manner.

Owing to the close relationship between a free compound, a prodrug derivative and a salt compound, a certain compound in this invention also encompasses its prodrug derivative and salt form, where this is possible and appropriate. A reference to a compound of general formula (I), independently of any given specifc form, is therefore also to be understood as implicitly including the proddrug derivatives and pharmaceutically usable salts thereof.

The compounds of the formula (I) also include those compounds in which one or more atoms are replaced by their stable, non-radioactive isotopes; for example, a hydrogen atom by deuterium.

The compounds of formula (I) and (Ia), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 ul buffer, 10 ul inhibitor in DMSO, 10 ul peptide substrate in DMSO and 20 ul enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems eg the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

### In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compound groups mentioned below are not to be regarded as closed, but rather parts of these compound groups may be exchanged with one another or with the definitions given above or omitted in a sensible manner, for example to replace general by more specific definitions.

Preference is given to compounds of the formula (I) in which X is methylene.

Preference is given to compounds of the formula (I) in which R¹ is hydrogen or C₁-C₈-alkyl.

When R² is aryl-C₀-C₄-alkyl, it is preferably aryl-C₀-C₁-alkyl.

Preference is given to compounds of the formula (I), in which R² is C₁-C₈-alkyl, C₃-C₈-cycloalkyl, C₁-C₈-alkylsulphonyl, C₃-C₈-cycloalkylsulphonyl, aryl-C₀-C₈-alkylsulphonyl, C₃-C₁₂-cycloalkyl-C₁-C₈-alkanoyl, C₃-C₁₂-cycloalkyl-C₃-C₈-cycloalkanoyl, aryl-C₁-C₈-alkanoyl, heterocyclyl-C₁-C₈-alkanoyl, C₁-C₈-alkanoyl or aryl-C₀-C₄-alkyl, which radicals may be substituted by 1-4 C₁-C₈-alkyl, C₃-C₈-cycloalkyl, C₃-C₈-cycloalkoxy, C₀-C₆-alkylcarbonylamino, halogen, cyano, hydroxyl, oxide, trifluoromethyl, C₁-C₈-alkoxy or optionally N-mono- or N,N-di-C₁-C₈-alkylated carbamoyl.

Particular preference is given to compounds of the formula (I) in which R² is C₃-C₈-cycloalkyl, C₁-C₈-alkylsulphonyl, C₃-C₈-cycloalkylsulphonyl, aryl-C₀-C₈-alkylsulphonyl, C₃-C₁₂cycloalkyl-C₁-C₈-alkanoyl, C₃-C₁₂-cycloalkyl-C₃-C₈-cycloalkanoyl, aryl-C₁-C₈-alkanoyl, heterocyclyl-C₁-C₈-alkanoyl or C₁-C₈-alkanoyl, which radicals may be substituted by 1-4 C₁-C₈-alkyl, C₃-C₈-cycloalkyl, C₃-C₈-cycloalkoxy, C₀-C₆-alkylcarbonylamino, halogen, cyano, hydroxyl, oxide, trifluoromethyl, C₁-C₈-alkoxy or optionally N-mono- or N,N-di-C₁-C₈-alkylated carbamoyl.

Very particular preference is given to compounds of the formula (I) in which R² is C₁-C₈-alkylsulphonyl, C₃-C₈-cycloalkylsulphonyl, aryl-C₀-C₈-alkylsulphonyl, C₃-C₁₂-cycloalkyl-C₁-C₈-alkanoyl, C₃-C₁₂-cycloalkyl-C₃-C₈-cycloalkanoyl, aryl-C₁-C₈-alkanoyl, heterocyclyl-C₁-C₈-alkanoyl or C₁-C₈-alkanoyl, which radicals may be substituted by 1-4 C₁-C₈-alkyl, C₃-C₈-cycloalkyl, C₃-C₈-cycloalkoxy, C₀-C₆-alkylcarbonylamino, halogen, cyano, hydroxyl, oxide, trifluoromethyl, C₁-C₈-alkoxy or optionally N-mono- or N,N-di-C₁-C₈-alkylated carbamoyl.

Preference is further given to compounds of the formula (I) in which R² together with R¹ and the nitrogen atom to which they are bonded, is a saturated or partly unsaturated, 4-8-membered heterocyclic ring which may contain an additional nitrogen or oxygen atom, in which case the additional nitrogen atom may optionally be substituted by C₁-C₈-alkyl or C₁-C₈-alkanoyl, in which case this heterocyclic ring may be part of a bicyclic or tricyclic ring system having a total of up to 16 ring members and the second ring may also contain a nitrogen or oxygen atom, and the nitrogen atom of the second ring may optionally be substituted by C₁-C₈-alkyl or C₁-C₈-alkanoyl, and all ring systems mentioned may be substituted by 1-4 C₁-C₈-alkyl, hydroxyl, oxide, oxo, C₁-C₈-alkoxy, C₁-C₈-alkoxy-C₁-C₈-alkoxy, C₁-C₈-alkanoylamino or aryloxy-C₀-C₄-alkyl-C₁-C₈-alkoxy.

Preference is further given to compounds of the formula (I) in which, when X is methylene,
R¹
a) is hydrogen; or
b) is C₁-C₈-alkyl or C₃-C₈-cycloalkyl;
R²
a) is C₁-C₈-alkyl, C₃-C₈-cycloalkyl, C₁-C₈-alkanoyl, heterocyclyl-C₁-C₈-alkanoyl, C₃-C₁₂-cycloalkyl-C₁-C₈-alkanoyl or aryl-C₁-C₈-alkanoyl, which radicals may be substituted by 1-4 C₁-C₈-alkyl, C₁₋₆-alkylamino, cyano, halogen, hydroxyl, C₁-C₆-alkanoylamino, C₁-C₈-alkoxy, oxide, oxo, trifluoromethyl or aryl; or
b) together with R¹ and the nitrogen atom to which they are bonded, is a saturated or partly unsaturated, 4-8-membered heterocyclic ring which may contain an additional nitrogen or oxygen atom, in which case the additional nitrogen atom may optionally be substituted by C₁-C₈-alkyl or C₁-C₈-alkanoyl, in which case this heterocyclic ring may be part of a bicyclic or tricyclic ring system having a total of up to 16 ring members and the second ring may also contain a nitrogen or oxygen atom, and the nitrogen atom of the second ring may optionally be substituted by C₁-C₈-alkyl or C₁-C₈-alkanoyl, and all ring systems mentioned may be substituted by 1-4 C₁-C₈-alkyl, hydroxyl, oxide, oxo, C₁-C₈-alkoxy, C₁-C₈-alkoxy-C₁-C₈-alkoxy, C₁-C₈-alkanoylamino or aryloxy-C₀-C₄-alkyl-C₁-C₈-alkoxy.

The invention further preferably relates to compounds of the formula (I) in which
X is methylene;
R¹ a) is hydrogen; or
b) is C₁-C₈-alkyl or C₃-C₈-cydoalkyl;
   R² a) is C₁-C₈-alkyl, C₃-C₈-cydoalkyl, C₁-C₈-alkanoyl, heterocyclyl-C₁-C₈-alkanoyl, C₃-C₁₂-cycloalkyl-C₁-C₈-alkanoyl or aryl-C₁-C₈-alkanoyl, which radicals may be substituted by 1-4 C₁-C₈-alkyl, C₁₋₆-alkylamino, cyano, halogen, hydroxyl, C₁-C₆-alkanoylamino, C₁-C₈-alkoxy, oxide, oxo, trifluoromethyl or aryl; or
b) together with R¹ and the nitrogen atom to which they are bonded, is a saturated or partly unsaturated, 4-8-membered heterocyclic ring which may contain an additional nitrogen or oxygen atom, in which case the additional nitrogen atom may optionally be substituted by C₁-C₈-alkyl or C₁-C₈-alkanoyl, in which case this heterocyclic ring may be part of a bicyclic or tricyclic ring system having a total of up to 16 ring members and the second ring may also contain a nitrogen or oxygen atom, and the nitrogen atom of the second ring may optionally be substituted by C₁-C₈-alkyl or C₁-C₈-alkanoyl, and all ring systems mentioned may be substituted by 1-4 C₁-C₈-alkyl, hydroxyl, oxide, oxo, C₁-C₈-alkoxy, C₁-C₈-alkoxy-C₁-C₈-alkoxy, C₁-C₈-alkanoylamino or aryloxy-C₀-C₄-alkyl-C₁-C₈-alkoxy;
   R³ is hydrogen;
   R⁴ is hydrogen;
   R⁵ are each independently hydrogen or C₁-C₈-alkyl; and
   R⁶ is as defined in the above-specified groups (A) and (B)
   and pharmaceutical usable salts thereof.

Particular preference is further given to compounds of the formula (I) in which one R⁵ radical is hydrogen and one R⁵ radical is C₁-C₈-alkyl.

Especially preferred R⁶ radicals are furyl, thienyl, pyridyl, pyrimidyl, indolyl, quinolinyl, benzoimidazolyl, di-C₁₋₆-alkoxypyrimidinyl, 2- and 5-benzo[b]thienyl, 6- and 7-isoquinolyl, 6- and 7-tetrahydroquinolyl, 6- and 7-tetrahydroisoquinolyl, 6-quinoxalinyl, 6- and 7-quinazolinyl, dihydro-3H-benzo[1,4]oxazinyl, 3,4-dihydro-2H-benzo[1,4]oxazinyl, 3-oxo-4H-benzo[1,4]oxazinyl, 2-oxobenzooxazolyl, 2-oxo-1,3-dihydroindolyl, 2,3-dihydroindolyl, indazolyl or benzofuranyl;
and 6- and 7-quinolyl, 6- and 7-isoquinolyl, 6- and 7-tetrahydroquinolyl, oxotetrahydroquinolyl, 6- and 7-tetrahydroisoquinolyl, 6-quinoxalinyl, 6- and 7-quinazolinyl, indolyl, dihydro-3H-benzo[1,4]oxazinyl, 3,4-dihydro-2H-benzo[1,4]oxazinyl, 3-oxo-3,4-dihydro-2H-benzo[1,4]oxazinyl, 3-oxo-4H-benzo[1,4]oxazinyl, 2-oxobenzooxazolyl, 2-oxo-2,3-dihydrobenzooxazolyl, 2-oxo-1,3-dihydroindolyl, 2,3-dihydroindolyl, indazolyl, benzofuranyl, 2,3-dihydrobenzothiazinyl, imidazolyl, benzimidazolyl, pyridinyl, pyrrolo [2,3-b]pyridinyl, pyrrolo[3,2-c]pyridinyl, pyrrolo[2,3-c]pyridinyl, pyrrolo[3,2-b]pyridinyl, [1,2,3]triazolo[1,5-a]pyridinyl, [1,2,4]triazolo[4,3-a]pyridinyl, imidazo[1,2-a]pyrimidinyl, imidazo[1,5-a]pyridinyl, naphthyl or cyclohexenophenyl, each of which is substituted by from one to four radicals selected from C₁₋₆-alkyl, cyano, oxo, oxide, trifluoromethyl, hydroxyl, halogen, carbamoyl, carboxy, C₁₋₆-alkoxy, hydroxy-C₂₋₇-alkoxy, C₁₋₆-alkoxy-C₁₋₆-alkoxy, di-C₁₋₆-alkylamino, 2,3-dihydroxypropoxy, 2,3-dihydroxypropoxy-C₁₋₆-alkoxy, 2,3-dimethoxypropoxy, methoxybenzyloxy, hydroxybenzyloxy, phenethyloxy, methylenedioxybenzyloxy, dioxolanyl-C₁₋₆-alkoxy, cyclopropyl-C₁₋₆-alkoxy, pyridylcarbamoyloxy-C₁₋₆-alkoxy, 3-morpholino-2-hydroxypropoxy, benzyloxy-C₁₋₆-alkoxy, picolyloxy, C₁₋₆-alkoxycarbonyl, C₁₋₆-alkoxy-C₁₋₆-alkoxy-C₁₋₆-alkyl, C₁₋₆-alkylcarbonylamino, C₁₋₆-alkylcarbonylamino-C₁₋₆-alkyl, C₁₋₆-alkylcarbonylamino-C₁₋₆-alkoxy, (N-C₁₋₆-alkyl)-C₁₋₆-alkylcarbonylamino-C₁₋₆-alkyl, (N-C₁₋₆-alkyl)-C₁₋₆-alkylcarbonylamino-C₁₋₆-alkoxy, C₃₋₆-cycloalkylcarbonylamino-C₁₋₆-alkyl, C₃₋₆-cycloalkylcarbonylamino-C₁₋₆-alkoxy, C₁₋₆-alkoxy-C₁₋₆-alkyl, hydroxy-C₁₋₆-alkyl, hydroxy-C₂₋₇-alkoxy-C₁₋₆-alkyl, hydroxy-C₂₋₇-alkoxy-C₁₋₆-alkoxy, C₁₋₆-alkoxycarbonylamino-C₁₋₆-alkyl, C₁₋₆-alkoxycarbonylamino-C₂₋₇-alkoxy, C₁₋₆-alkylaminocarbonylamino-C₁₋₆-alkyl, C₁₋₆-alkylaminocarbonylamino-C₂₋₇-alkoxy, C₁₋₆-alkylaminocarbonyl-C₁₋₆-alkyl, C₁₋₆-alkylaminocarbonyl-C₁₋₆-alkoxy, C₁₋₆-alkylaminocarbonyl-C₁₋₆-alkoxy-C₁₋₆-alkyl, di-C₁₋₆-alkylaminocarbonyl-C₁₋₆-alkyl, di-C₁₋₆-alkylaminocarbonyl-C₁₋₆-alkoxy, C₁₋₆-alkylcarbonyloxy-C₁₋₆-alkyl, C₁₋₆-alkylcarbonyloxy-C₁₋₆-alkoxy, cyano-C₁₋₆-alkyl, cyano-C₁₋₋₆-alkoxy, 2-oxooxazolidinyl-C₁₋₆-alkyl, 2-oxooxazolidinyl-C₁₋₆-alkoxy, C₁₋₆-alkoxycarbonyl-C₁₋₆-alkyl, C₁₋₆-alkoxycarbonyl-C₁₋₆-alkoxy, C₁₋₆-alkylsulphonylamino-C₁₋₆-alkyl, C₁₋₆-alkylsulphonylamino-C₂₋₇-alkoxy, (N-C₁₋₆-alkyl)-C₁₋₆-alkylsulphonylamino-C₁₋₆-alkyl, (N-C₁₋₆-alkyl)-C₁₋₆-alkylsulphonylamino-C₁₋₆-alkoxy, C₁₋₆-alkylamino-C₁₋₆-alkyl, C₁₋₆-alkylamino-C₂₋₇-alkoxy, di-C₁₋₆-alkylamino-C₁₋₆-alkyl, Di-C₁₋₆-alkylamino-C₂₋₇-alkoxy, C₁₋₆-alkylsulphonyl-C₁₋₆-alkyl, C₁₋₆-alkylsulphonyl-C₁₋₆-alkoxy, carboxy-C₁₋₆-alkyl, carboxy-C₁₋₆-alkoxy, carboxy-C₁₋₆-alkoxy-C₁₋₆-alkyl, C₁₋₆-alkoxy-C₁₋₆-alkylcarbonyl, acyl-C₁₋₆-alkoxy-C₁₋₆-alkyl, (N-C₁₋₆-alkyl)-C₁₋₆-alkoxycarbonylamino, (N-hydroxy)-C₁₋₆-alkylaminocarbonyl-C₁₋₆-alkyl, (N-hydroxy)-C₁₋₆-alkylaminocarbonyl-C₁₋₆-alkoxy, (N-hydroxy)aminocarbonyl-C₁₋₆-alkyl, (N-hydroxy)aminocarbonyl-C₁₋₆-alkoxy, C₁₋₆-alkoxyaminocarbonyl-C₁₋₆-alkyl, 6-alkoxy-aminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆-alkylaminocarbonyl-C₁₋₆-alkoxy, (N-acyl)-C₁₋₆-alkoxy-C₁₋₆-alkylamino, C₁₋₆-alkoxy-C₁₋₆-alkylcarbamoyl, (N-C₁₋₆-alkyl)-C₁₋₆-alkoxy-C₁₋₆-alkylcarbamoyl, C₁₋₆-alkoxy-C₁₋₆-alkylcarbonyl, C₁₋₆-alkoxy-C₁₋₆-alkylcarbonylamino, (N-C₁₋₆-alkyl)-C₁₋₆-alkoxy-C₁₋₆-alkylcarbonylamino, 1-C₁₋₆-alkoxy-C₁₋₆-alkylimidazol-2-yl, 1-C₁₋₆-alkoxy-C₁₋₆-alkyltetrazol-5-yl, 5-C₁₋₆-alkoxy-C₁₋₆-alkyltetrazol-1-yl, 2-C₁₋₆-alkoxy-C₁₋₆-alkyl-4-oxoimidazol-1-yl, carbamoyl-C₁₋₆-alkyl, carbamoyl-C₁₋₆-alkoxy, C₁₋₆-alkylcarbamoyl, di-C₁₋₆-alkylcarbamoyl, C₁₋₆-alkylsulphonyl, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyl-tetrazol-1-ylalkyl, 5-methyl-tetrazol-1-ylalkoxy, thiazol-4-ylalkyl, thiazol-4-ylalkoxy, oxazol-4-ylalkyl, oxazol-4-ylalkoxy, 2-oxo-pyrrolidinylalkyl, 2-oxo-pyrrolidinylalkoxy, imidazolylalkyl, imidazolylalkoxy, 2-methyl-imidazolylalkyl, 2-methyl-imidazolylalkoxy, N-methylpiperazinoalkyl, N-methylpiperazinoalkoxy, N-methylpiperazinoalkoxyalkyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₆-alkoxy-C₁₋₆-alkyl-pyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl and 2-oxotetrahydropyrimidinyl.

Further especially preferred R⁶ radicals are heterocyclyl radicals, preferably indolyl, 3,4-dihydro-2H-benzo[1,4]oxazinyl, indazolyl, benzofuranyl, benzoimidazolyl, pyridinyl, pyrrolo[2,3-b]pyridinyl, pyrrolo[3,2-c]pyridinyl, pyrrolo[2,3-c]pyridinyl, pyrrolo[3,2-b]pyridinyl, [1,2,3]triazolo[1,5-a]pyridinyl, [1,2,4]triazolo[4,3-a]pyridinyl, imidazo[1,2-a]pyrimidinyl or imidazo[1,5-a]pyridinyl, or naphthyl, each of which is substituted by from one to four radicals selected from C₁₋₆-alkyl, cyano, oxo, oxide, trifluoromethyl, hydroxyl, halogen, carbamoyl, carboxy, C₁₋₆-alkoxy, hydroxy-C₂₋₇-alkoxy, C₁₋₆-alkoxy-C₁₋₆-alkoxy, C₁₋₆-alkoxy-C₁₋₆-alkyl or C₁₋₆-alkoxy-C₁₋₆-alkoxy-C₁₋₆-alkyl.

Particular preference is given in each case to those compounds of the formula (I) in which at least one asymmetric carbon atom, for example one, two or preferably all three asymmetric carbon atoms, of the main chain have the stereochemistry (in each case "S") shown in the formula (Ia) 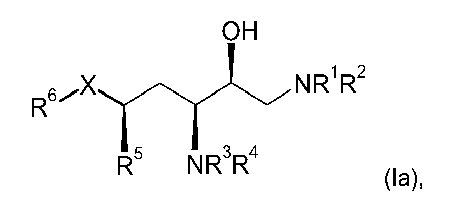 or a pharmaceutically usable salt thereof; wherein the substituents are each as defined above including the preferences specified.

The compounds of the formula (I) or formula (Ia) or a pharmaceutically usable salt thereof may be prepared in an analogous manner to preparative processes known from the literature. The starting materials to carry out the preparative processes are described, for example, in EP 0678503 and in Helvetica Chemica Acta 86 (2003), 2848-2870 or literature cited therein. The inventive compounds of the formula I and salts of such compounds having at least one salt-forming group are obtained by processes known per se, for example by
a) condensing a compound of the formula II 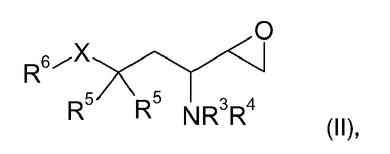 wherein X, R³, R⁴, R⁵ and R⁶ are each as defined above or a salt thereof with a compound of the formula R¹R²NH (III) where R¹ and R² are each as defined above, in the course of which free functional groups in the reaction components with the exception of the groups taking part in the reaction are present in protected form, and detaching protecting groups present. In cases where R¹ and R² are a saturated or partly unsaturated oxo-substituted heterocyclic ring (for example lactams) and strong bases are used as a reagent, the alkoxide formed by epoxide formation may react with one of the protecting groups present (e.g. N-Boc) and form an oxazolidinone which may be cleaved to give the product, for example with lithium hydroxide, or
b) condensing a compound of the formula II 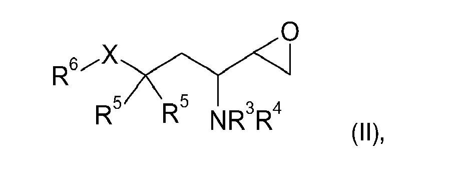 wherein X, R³, R⁴, R⁵ and R⁶ are each as defined above or a salt thereof with an azide, reducing the azido group to amino and then, depending on the definitions of R¹ and R², mono- or dialkylating, mono- or diacylating, and also optionally sulphonylating the amino group, in the course of which free functional groups present in the reaction components with the exception of the groups taking part in the reaction are present in protected form, and detaching protecting groups present, or
c) condensing a compound of the formula IV 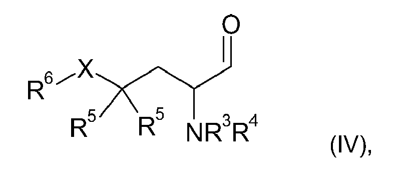
wherein X, R³, R⁴, R⁵ and R⁶ are each as defined above or a salt thereof with cyanide or nitromethane, reducing the nitrile group or nitro group to amino, and then, depending on the definitions of R¹ and R², mono- or dialkylating, mono- or diacylating, and also optionally sulphonylating the amino group, in the course of which free functional groups present in the reaction components with the exception of the groups taking part in the reaction are present in protected form, and detaching protecting groups present.

Compounds of the formula II can be prepared in an analogous manner to preparative processes known from the literature, for example by
a) condensing a compound of the formula IV 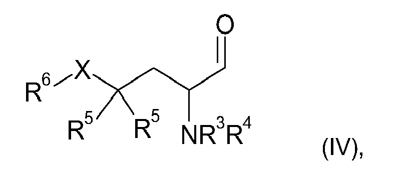 wherein X, R³, R⁴, R⁵ and R⁶ are each as defined above or a salt thereof with methylide (see, for example, in Tet. Lett. 30(40), 5425-5428, 1989), in the course of which free functional groups present in the reaction components with the exception of the groups taking part in the reaction are present in protected form, and detaching protecting groups present, or
b) epoxidizing a compound of the formula V 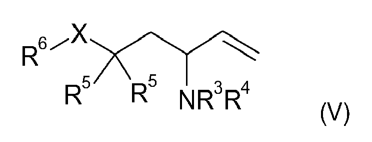 wherein X, R³, R⁴, R⁵ and R⁶ are each as defined above or a salt thereof (see, for example, in J. Med. Chem. 35(10), 1685-1701, 1992 and J. Org. Chem. 59(3), 653-657, 1994), in the course of which free functional groups present in the reaction components with the exception of the groups taking part in the reaction are present in protected form, and protecting groups present are detached, or
c) dihydroxylating a compound of the formula V 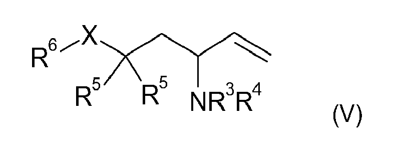 wherein X, R³, R⁴, R⁵ and R⁶ are each as defined above or a salt thereof, tosylating the primary alcohol and subsequently admixing with a base such as potassium hydroxide (see, for example in WO 03050073), in the course of which free functional groups present in the reaction components with the exception of the groups taking part in the reaction are present in protected form, and detaching protecting groups present, or
d) preparing an activated ester from a compound of the formula VI 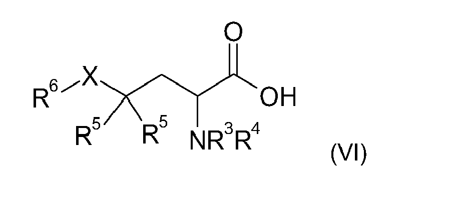
wherein X, R³, R⁴, R⁵ and R⁶ are each as defined above or a salt thereof and admixing it with diazomethane, admixing the diazoketone with 48% HBr, and then reducing the bromoketone and subsequently admixing it with a base such as potassium hydroxide (see, for example, in WO 03050073), in the course of which free functional groups present in the reaction components with the exception of the groups taking part in the reaction are present in protected form, and detaching protecting groups present.

Details of the specific preparation variants can be taken from the examples.

Depending on the presence of asymmetric carbon atoms, the inventive compounds may be in the form of isomer mixtures, especially as racemates, or in the form of pure isomers, especially of optical antipodes.

The compounds of the formula (I) may also be prepared in optically pure form. The separation into antipodes may be effected by methods known per se, either preferably at a synthetically early stage by salt formation with an optically active acid, for example (+)- or (-)-mandelic acid and separation of the diastereomeric salts by fractional crystallization, or preferably at a rather later stage by derivatization with a chiral auxiliary building block, for example (+)- or (-)-camphanoyl chloride, and separation of the diastereomeric products by chromatography and/or crystallization and subsequent cleavage of the bond to the chiral auxiliary. To determine the absolute configuration of the piperidine present, the pure diastereomeric salts and derivatives may be analysed with common spectroscopic methods, of which X-ray spectroscopy on single crystals constitutes a particularly suitable method.

The compounds of the formula (I) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (la), respectively, and their pharmaceutically useful salts for the prevention, delay of progression or the treatment of Alzheimer Disease, malaria or HIV infection, preferably for the prevention, delay of progression or the treatment of malaria or HIV infection.

Subject of the present invention is also the use of the compounds of formula (I) and (la), respectively, and their pharmaceutically useful salts for the manufacture of a medication for the prevention, delay of progression or the treatment of Alzheimer Disease, malaria or HIV infection, preferably for the manufacture of a medication for the prevention, delay of progression or the treatment of malaria or HIV infection.

Subject of the present invention is also the method for the prevention, delay of progression or the treatment of Alzheimer Disease, malaria or HIV infection, preferably the method for the prevention, delay of progression or the treatment of malaria or HIV infection, whereby a therapeutically effective dose of a compound of the general formula (I) or preferred formula (Ia) or a pharmaceutically usable salt thereof is applied.

Subject of the present invention is also a pharmaceutical preparation that contains for the inhibition of beta-secretase, cathepsin D, plasmepsin and/or HIV-protease a compound of the general formula (I) or a pharmaceutically usable salt thereof, or preferred of formula (la) or a pharmaceutically usable salt thereof as well as commonly used ingredients.

Subject of the present invention is also a pharmaceutical preparation for the prevention, delay of progression or treatment of Alzheimer Disease, malaria and HIV infection, preferably for the prevention, delay of progression or treatment of malaria and HIV infection, that contains a compound of the general formula (I) or a pharmaceutically usable salt thereof, or preferred of formula (la) or a pharmaceutically usable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is determined in the solvent system A. The ratio of solvents relative to one another is always reported in parts by volume. Chemical names for end products and intermediates were generated with the aid of the program AutoNom 2000 (automatic nomenclature). Unless stated otherwise, the absolute stereochemistry of all three asymmetric carbon atoms of the main chain of the formula (Ia) is in each case "S".

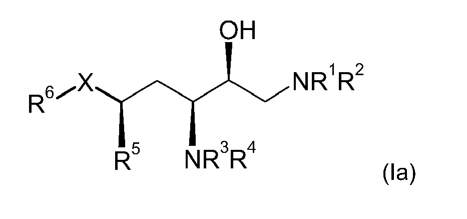

The Example Compounds 1A to 1QQQ correspond to the formula (Ib) 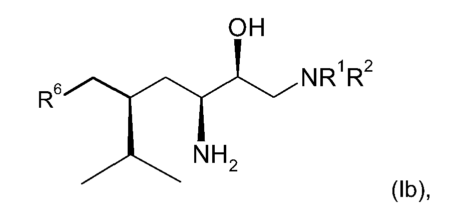 wherein R⁶ and NR¹R² correspond to the below-specified radicals which are prepared as described in detail in WO2005/090304, pages 28 to 47 and 48 to 62, respectively, which description is herewith incorporated. R⁶ and NR¹R² in each example compound 1A to 1QQQ corresponds to one of the below-specified residues A to QQQ. The atoms denoted by * are the bonding sites. The further Example Compounds 2A to 41 QQQ are accordingly the compounds of formula (Ib) in which the NR¹R² radical assumes all above residue-definitions (A to QQQ) for a given R⁶ (above residue-definitions 2 to 41). Thus, example compound 3A is the compound 3-Amino-1-isopropylamino-5-[3-(3-methoxypropyl)-1-methyl-1H-indol-5-ylmethyl]-6-methylheptan-2-ol dihydrochloride.

Analogously to that preparation processes, the remaining compounds 1 A to 41 QQQ are obtained.

Details of specific preparation variants can be taken from the examples.

HPLC gradients on Hypersil BDS C-18 (5 µm); column: 4 x 125 mm
- I: 90% water*/10% acetonitrile* to 0% water*/100% acetonitrile* in 5 minutes + 2.5 minutes (1.5 ml/min)
- II: 95% water*/5% acetonitrile* to 0% water*/100% acetonitrile* in 40 minutes (0.8 ml/min)
- *: contains 0.1 % trifluoroacetic acid

The following abbreviations are used:
- Rf: ratio of distance travelled by a substance to separation of the eluent front from the start point in thin-layer chromatography
- Rt: retention time of a substance in HPLC (in minutes)
- m.p.: melting point (temperature)
- b.p.: boiling point (temperature)

General methods used are described in detail in WO2005/090304 pages 27 to 28, which description is herewith incorporated

The following examples are prepared as described in detail in WO2005/090304 pages 63 to 73 which description is herewith incorporated.

### Example compound 3A:

### 3-Amino-1-isopropylamino-5-[3-(3-methoxy-propyl)-1-methyl-1H-indol-5-ylmethyl]-6-methylheptan-2-ol dihydrochloride

### Example compound 35G:

### 3-Amino-5-[8-(2-methoxy-ethoxy)-naphthalen-2-ylmethyl]-6-methyl-1-piperidin-1-yl-heptan-2-ol dihydrochloride

### Example compound 3J:

### 1-{3-Amino-2-hydroxy-5-[3-(3-methoxy-propyl)-1-methyl-1H-indol-5-ylmethyl]-6-methylheptyl}-piperidin-2-one hydrochloride

### Example compound 3K:

### N-{3-Amino-2-hydroxy-5-[3-(3-methoxy-propyl)-1-methyl-1H-indol-5-ylmethyl]-6-methylheptyl}-2,2-dimethyl-propionamide hydrochloride

### Example compound 3WW:

### Propan-2-sulphonic acid {3-amino-2-hydroxy-5-[3-(3-methoxy-propyl)-1-methyl-1H-indol-5-ylmethyl]-6-methyl-heptyl}-amide hydrochloride

Radicals R⁶ :

| | | | |
|---|---|---|---|
| 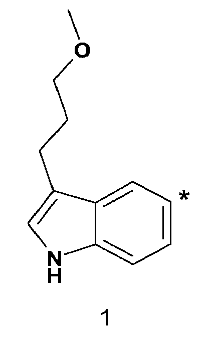 | 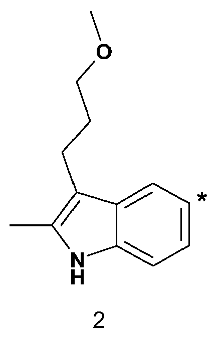 | 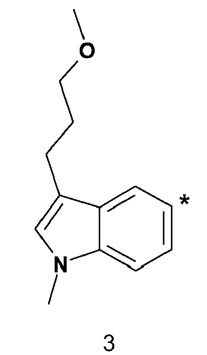 | 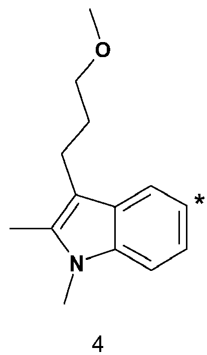 |
| 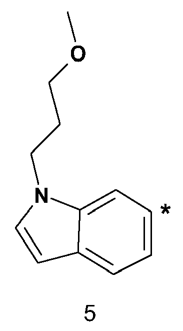 | 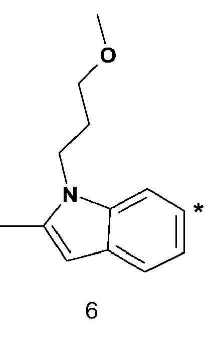 | 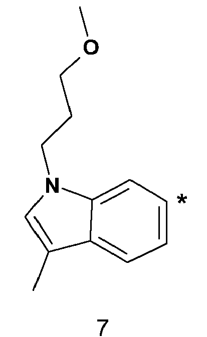 | 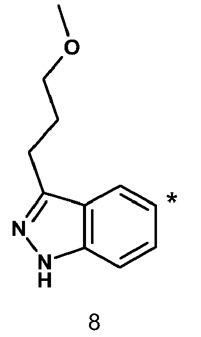 |
| 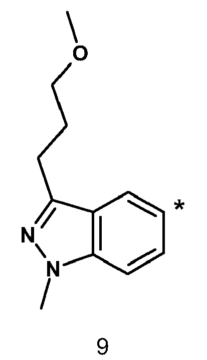 | 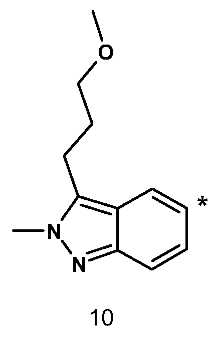 | 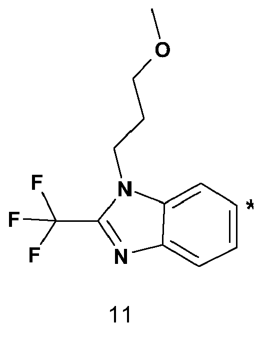 | 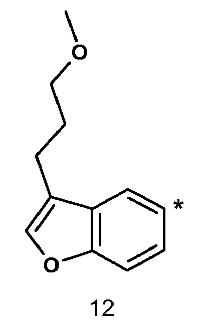 |
| 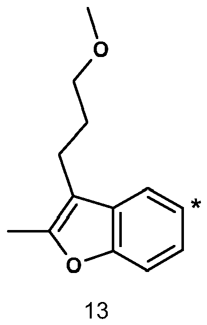 | 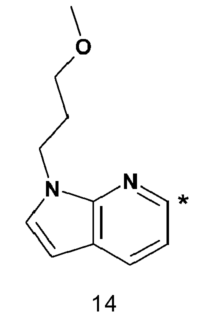 | 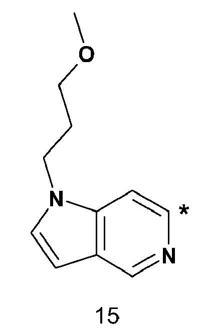 | 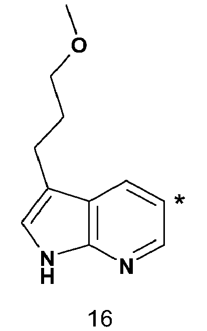 |
| 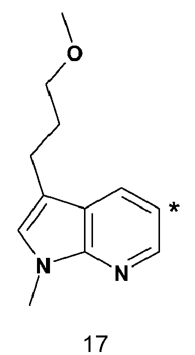 | 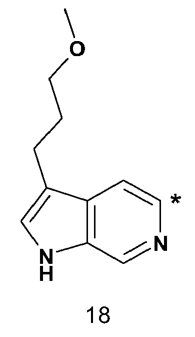 | 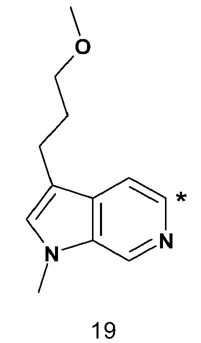 | 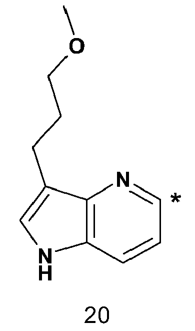 |
| 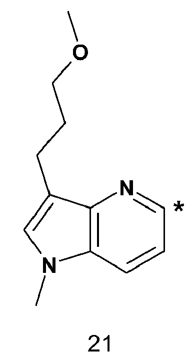 | 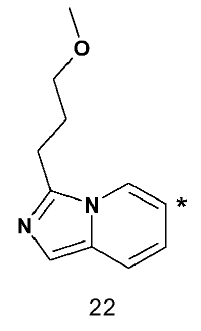 | 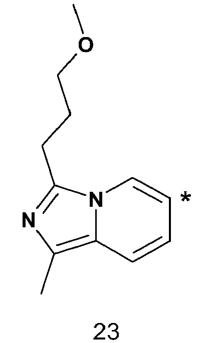 | 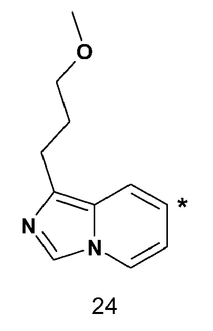 |
| 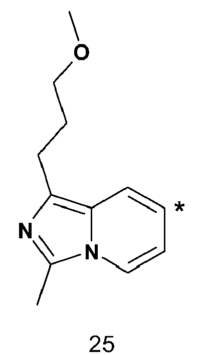 | 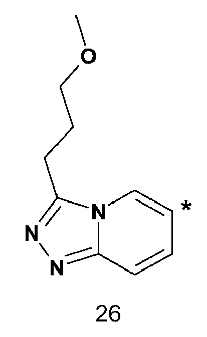 | 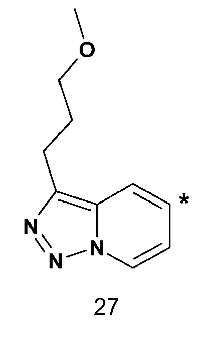 | 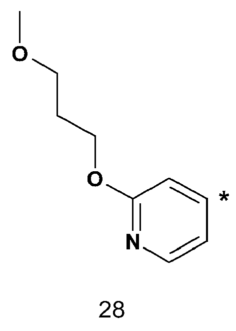 |
| 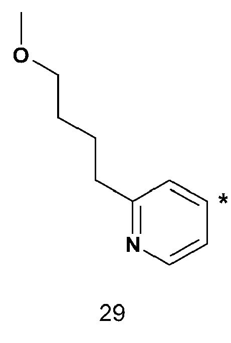 | 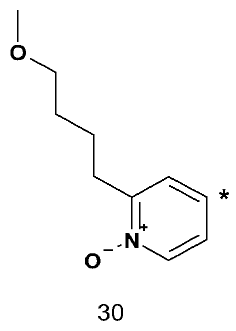 | 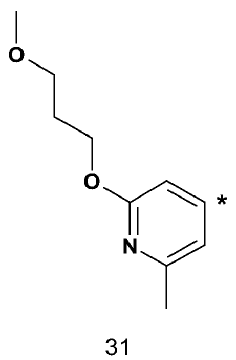 | 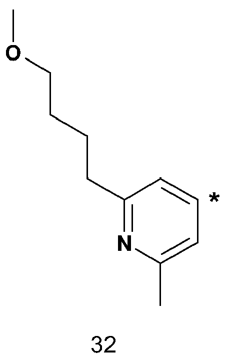 |
| 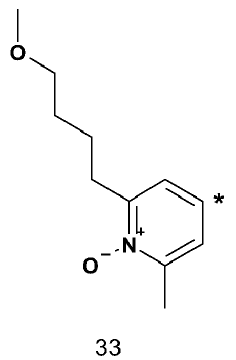 | 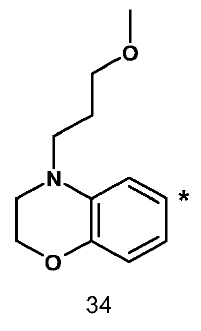 | 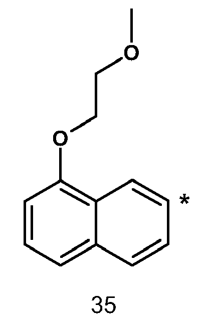 | 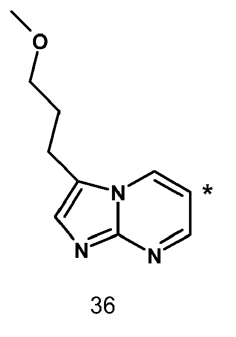 |
| 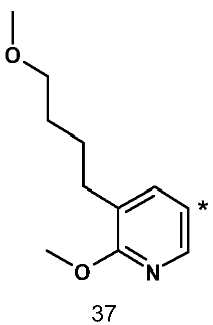 | 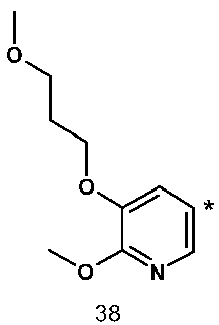 | 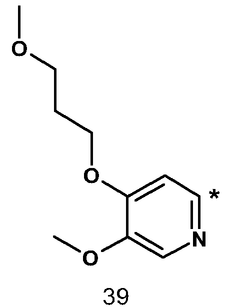 | 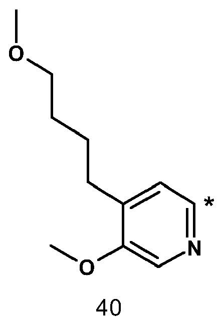 |
| 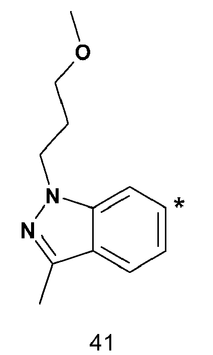 | | | |

### 22 6-Brom-3-(3-methoxypropyl)imidazo[1,5-a]pyridin

A solution of 8.02 g of 3-(6-bromo-imidazo[1,5-a]pyridin-3-yl)-propan-1-ol in 160 ml of N,N-dimethylformamide is treated with 1.38 g of sodium hydride (60% dispersion in oil) and stirred for 1 hour at room temperature. 2.5 ml of methyliodide are added and the reaction mixture is stirred for 2 hours at room temperature. The mixture is poured onto 1M sodium bicarbonate solution and extracted with dichloromethane (3x). The combined organic phases are dried over sodium sulphate and filtered, and the filtrate is concentrated by evaporation. The title compound is obtained from the residue as a yellow oil by means of flash chromatography (SiO₂ 60F). Rf = 0.46 (EtOAc); Rt = 2.69 (gradient I).

The starting materials are prepared as follows:

### a) 3-(6-Bromo-imidazo[1,5-a]pyridin-3-yl)-propan-1-ol

A solution of 9.62 g of 3-(6-bromo-imidazo[1,5-a]pyridin-3-yl)-propionic acid ethyl ester in 250 ml of tetrahydrofuran at -78°C is treated with 109 ml of DIBAL-H and warmed to room temperature over 1 hour. The mixture is poured onto a mixture of ice and 2M HCl and extracted with ethyl acetate (3x). The combined organic phases are dried over sodium sulphate and filtered, and the filtrate is concentrated by evaporation. The title compound is obtained as a brown oil from the residue by means of flash chromatography (SiO₂ 60F). Rf = 0.17 (dichloromethane-methanol-25% ammonia conc. 200:10:1); Rt = 2.19 (gradient I).

### b) 3-(6-Bromo-imidazo[1,5-a]pyridin-3-yl)-propionic acid ethyl ester

A solution of 15.59 g of N-(5-bromo-pyridin-2-ylmethyl)-succinamic acid ethyl ester in 170 ml of toluene is treated with 25 ml of phosphor oxychloride and heated for 10 hours to 60°C. Die The solution is cooled to 0°C and slowly treated with 2M NaOH till the ph Value is adjusted to 11. The solution is stirred for 45 minutes at room temperature and extracted with tert-butyl methyl ether (3x). The combined organic phases are dried over sodium sulphate and filtered, and the filtrate is concentrated by evaporation. The title compound is obtained as a yellow solid from the residue by means of flash chromatography (SiO₂ 60F). Rf = 0.34 (EtOAc-heptane 1:1); Rt = 2.93 (gradient I).

### c) N-(5-Bromo-pyridin-2-ylmethyl)-succinamic acid ethyl ester

A solution of 17.7 g of N-(5-bromo-pyridin-2-ylmethyl)-succinamic acid in 350 ml of ethanol is treated with 15.75 g of camphorsulphonic acid and heated to reflux for 3 hours. The solution is cooled to room temperature and 3 A molecular sieves are added. After 15 Minutes, the mixture is filtered, and the filtrate is concentrated by evaporation. The title compound is obtained as a white solid from the residue by means of flash chromatography (SiO₂ 60F). Rf = 0.22 (dichloromethane-methanol-25% ammonia conc. 200:10:1); Rt = 2.90 (gradient I).

### d) N-(5-bromo-pyridin-2-ylmethyl)-succinamic acid

A solution of 19.923 g of C-(5-bromo-pyridin-2-yl)-methylamine [173999-23-0] in 34 ml of pyridine is treated with 11.72 g of succinamic anhydride and stirred over night at room temperature. The white solid is collected by filtration, washed with little water and dried. Rt = 2.11 (gradient I).

### 23 6-Bromo-3-(3-methoxypropyl)-1-methylimidazo[1,5-a]pyridine

According to the method described for Residue 22, the title compound is obtained from 1-(5-bromo-pyridin-2-yl)-ethylamine.

The starting material is prepared as follows:

### a) 1-(5-Bromo-pyridin-2-yl)-ethylamine

A solution of 75 g of 5-bromo-pyridin-2-carbonitrile [97483-77-7] in 375 ml of tetrahydrofuran is treated dropwise with a solution of 172 ml methyl magnesium bromide (3M solution in diethyl ether) in 150 ml of tetrahydrofuran and stirred for Minutes at room temperature. The suspension is then treated with 750 ml of methanol and portionwise with 30 g of sodium borohydride. The reaction mixture is stirred 10 hours at room temperature, concentrated by evaporation and treated with ethyl acetate and 2m NaOH. The phases are separated and the aqueous phase is extracted with ethyl acetate (3x). The combined organic phases are washed with brine, dried over sodium sulphate and filtered, and the filtrate is concentrated by evaporation. The title compound is obtained as a brown oil from the residue by means of flash chromatography (SiO₂ 60F). Rf = 0.54 (dichloromethane-methanol-25% ammonia conc. 80:10:1); Rt = 2.02 (gradient I).

### 29 4-Brom-2-(4-methoxy-butyl)-pyridin

A solution of 3.17 mmol of 4-bromopyridine [1120-87-2] in 10 ml of tetrahydrofuran is cooled to -78°C and a solution of 3.49 mmol of 4-methoxybutylmagnesium chloride [634590-61-7] in 5 ml of diethyl ether is added. 3.17 mmol of phenyl chloroformate are added dropwise and the reaction mixture is stirred at -78°C for 10 minutes. Subsequently, the mixture is warmed to room temperature and quenched with 20% aqueous ammonium chloride solution. The phases of the filtrate are separated and the aqueous phase is extracted with diethyl ether. The combined organic phases are washed successively with water, 10% aqueous HCl, water and brine, dried over sodium sulphate and concentrated by evaporation. The residue is dissolved in 10 ml of toluene and 3.5 mmol of 3,4,5,6-tetrachloro-1,2-benzoquhinone and 7 ml of acetic acid are added. The reaction mixture is stirred at room temperature for 24 hours and basified with 10% aqueous NaOH. The mixture is stirred at 0°C for 15 minutes and filtered through Hyflo. The organic phase is extracted with 10% aqueous HCl (3x) - the combined aqueous phases are basified with 20% aqueous NaOH and extracted with dichloromethane (3x). The combined organic phases are dried over sodium sulphate and concentrated by evaporation. The title compound is obtained from the residue as a yellow oil by means of flash chromatography (SiO₂ 60F). Rf = 0.15 (EtOAc-heptane 1:3); Rt = 2.41 (gradient I).

### 30 4-Bromo-2-(4-methoxybutyl)pyridin-N-oxid

Example Compounds containing Residue 30 are obtained from precursors to Example Compounds containing Residue 29. In analogy to Example Compound 30VV, the oxidation to the N-oxide is carried out before the last step leading to the final example compound.

### 32 4-Bromo-2-(4-methoxybutyl)-6-methylpyridine

A solution of 6.54 g 4-(4-bromo-6-methyl-pyridin-2-yl)-butan-1-ol in 75 ml of N,N-dimethylformamide under Ar-atmosphere is cooled to 0°C. 1.25 g of sodium hydride (60% Dispersion in oil) is added portionwise over 15 minutes. The reaction mixture is stirred for 1 hour at room temperature and treated with 1.90 ml of methyl iodide. The reaction mixture is stirred for 16 hours, poured onto 1M sodium bicarbonate solution and extracted with tert-butyl methyl ether (2x). The combined organic phases are dried over sodium sulphate and filtered, and the filtrate is concentrated by evaporation. The title compound is obtained as a yellow oil from the residue by means of flash chromatography (SiO₂ 60F). Rf = 0.28 (EtOAc-heptane 1:1); Rt = 2.45 (gradient I).

The starting materials are prepared as follows:

### a) 4-(4-Brom-6-methyl-pyridin-2-yl)-butan-1-ol

A solution of 11.73 g 4-(4-bromo-6-methyl-pyridin-2-yl)-but-3-in-1-ol in 175 ml of ethanol is treated with 7.30 ml of triethylamine and 0.465 g platinum(II)oxide hydrate. The reaction mixture is hydrogenated for 2 hours at room temperature under atmospheric pressure. The reaction mixture is clarified by filtration and the filtrate is concentrated by evaporation. The title compound is obtained as a yellow oil from the residue by means of flash chromatography (SiO₂ 60F). Rf = 0.20 (EtOAc-heptane 3:1); Rt = 1.92 (gradient I).

### b) 4-(4-Bromo-6-methyl-pyridin-2-yl)-but-3-in-1-ol

A Mixture of 13.85 g of 2,4-dibromo-6-methyl-pyridine [79055-52-0], 1.80 g of bis(triphenylphosphine) palladium(II)chloride and 0.50 g of copper(I)iodide in 330 ml of triethylamine is treated with 4.30 ml of 3-butin-1-ol [927-74-2] under Ar atmosphere at 0°C. The reaction mixture is stirred for 4 hours at room temperature, diluted with water and extracted with tert-butyl methyl ether (2x). The combined organic phases are dried over sodium sulphate and filtered, and the filtrate is concentrated by evaporation. From the residue, the title compound is obtained as beige solid by re-crystallization from hot ethyl acetate-heptane 3:2. Rf = 0.15 (EtOAc-heptane 2:1); Rt = 2.51 (gradient I).

Radicals NR¹R²:

| | | | |
|---|---|---|---|
| 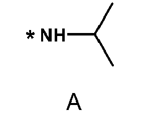 | 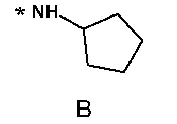 | 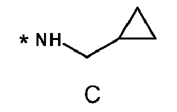 | 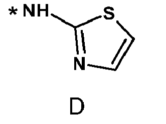 |
| 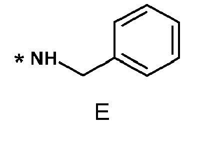 | 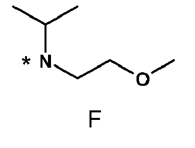 | 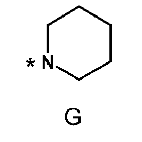 | 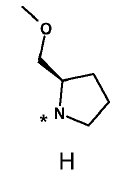 |
| 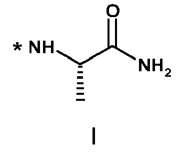 | 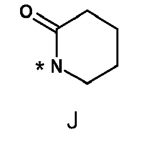 | 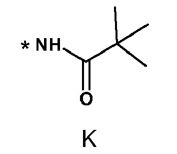 | 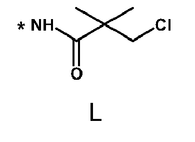 |
| 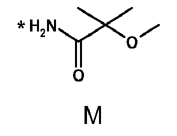 | 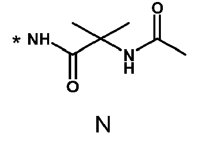 | 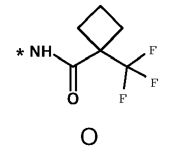 | 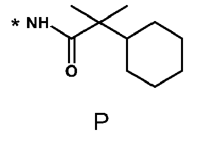 |
| 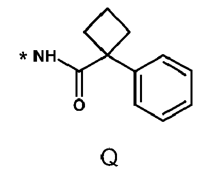 | 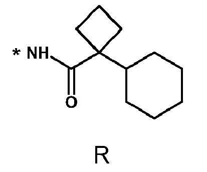 | 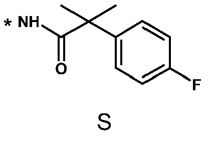 | 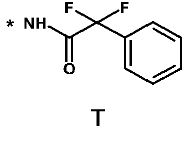 |
| 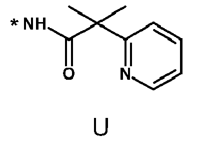 | 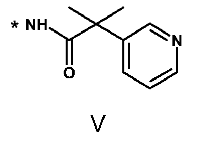 | 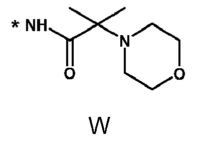 | 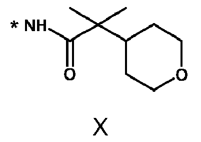 |
| 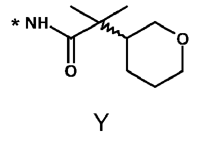 | 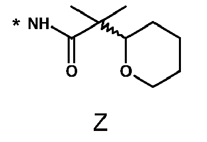 | 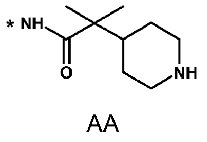 | 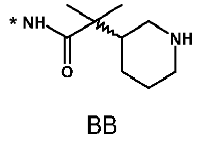 |
| 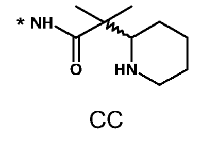 | 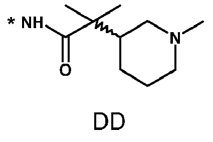 | 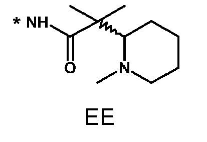 | 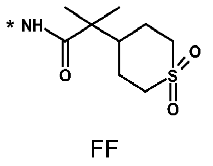 |
| 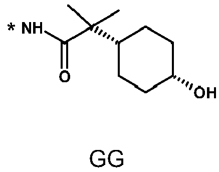 | 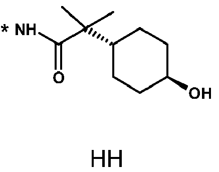 | 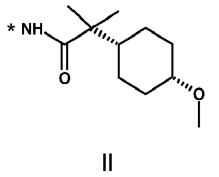 | 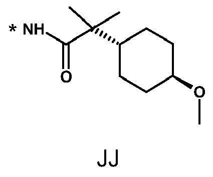 |
| 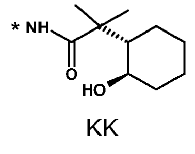 | 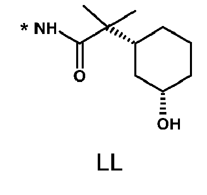 | 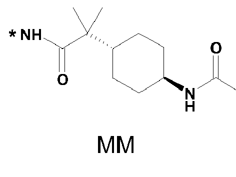 | 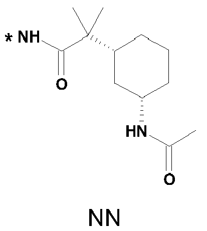 |
| 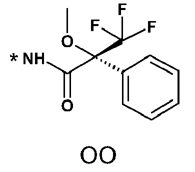 | 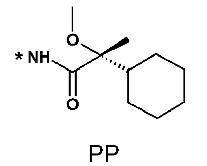 | 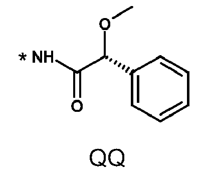 | 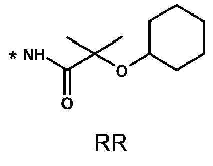 |
| 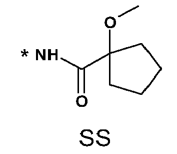 | 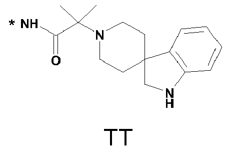 | 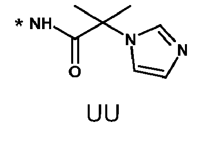 | 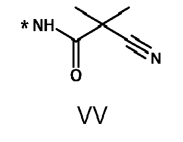 |
| 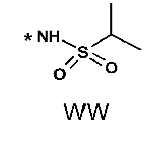 | 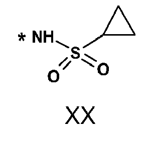 | 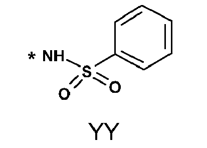 | 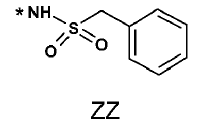 |
| 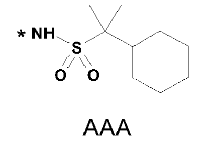 | 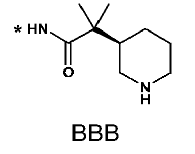 | 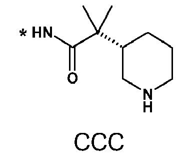 | 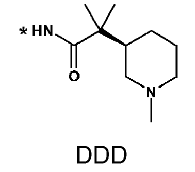 |
| 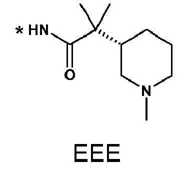 | 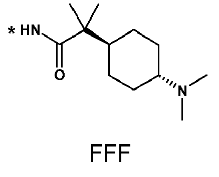 | 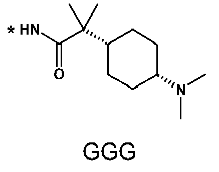 | 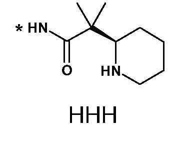 |
| 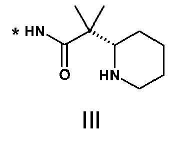 | 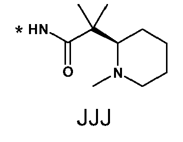 | 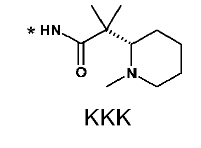 | 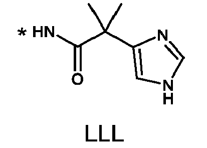 |
| 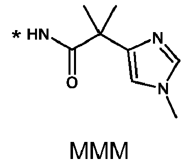 | 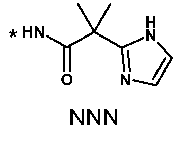 | 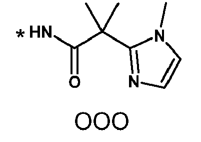 | 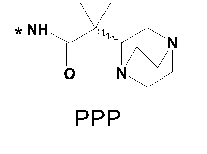 |
| 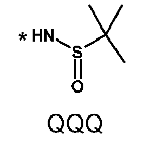 | | | |

## Claims

1. Use of a compound of formula 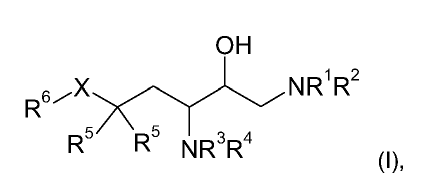 or a pharmaceutically usable salt thereof; wherein
X is methylene or hydroxymethylene;
R¹
a) is hydrogen; or
b) is C₁-C₈-alkyl, C₃-C₈-cydoalkyl, C₁-C₈-alkanoyl, C₁-C₈-alkoxycarbonyl, aryl-C₀-C₄-alkyl or heterocyclyl-C₀-C₄-alkyl, which radicals may be substituted by 1-4 C₁-C₈-alkyl, halogen, cyano, oxide, oxo, trifluoromethyl, C₁-C₈-alkoxy, C₁-C₈-alkoxycarbonyl, aryl or heterocyclyl; R² a) is C₁-C₈-alkyl, C₃-C₈-cydoalkyl, C₁-C₈-alkylsulphonyl, C₃-C₈-cydoalkylsulphonyl, aryl-C₀-C₈-alkylsulphonyl, heterocyclylsulphonyl, C₃-C₁₂-cycloalkyl-C₁-C₈-alkanoyl, C₃-C₁₂-cycloalkyl-C₃-C₈-cycloalkanoyl, aryl-C₁-C₈-alkanoyl, heterocydyl-C₁-C₈-alkanoyl, aryl-C₃-C₈-cycloalkanoyl, C₁-C₈-alkanoyl, C₁-C₈-alkoxycarbonyl, optionally N-mono or N,N-di-C₁-C₈-alkylated carbamoyl-C₀-C₈-alkyl, aryl-C₀-C₄-alkyl or heterocyclyl-C₀-C₄-alkyl, which radicals may be substituted by 1-4 C₁-C₈-alkyl, C₃-C₈-cydoalkyl, C₃-C₈-cydoalkoxy, amino, C₁₋₆-alkylamino, di-C₁₋₆-alkylamino, C₀-C₆-alkylcarbonylamino, halogen, cyano, hydroxyl, oxide, oxo, trifluoromethyl, C₁-C₈-alkoxy, optionally N-mono or N,N-di-C₁-C₈-alkylated carbamoyl, C₁-C₈-alkoxycarbonyl, C₁₋₆-alkylenedioxy, aryl or heterocyclyl; or
b) together with R₁ and the nitrogen atom to which they are bonded, is a saturated or partly unsaturated 4-8-membered heterocyclic ring which may contain an additional nitrogen, oxygen or sulphur atom or an -SO- or -SO2- group, and the additional nitrogen atom may optionally be substituted by C₁-C₈-alkyl, C₁-C₈-alkanoyl, C₁-C₈-alkoxycarbonyl, aryl or heterocyclyl radicals, in which case this heterocyclic ring may be part of a bicyclic or tricyclic ring system having a total of up to 16 members and the second ring may also contain a nitrogen, oxygen or sulphur atom or an -SO- or -SO2- group, and the nitrogen atom of the second ring may optionally be substituted by C₁-C₈-alkyl, C₁-C₈-alkanoyl, C₁-C₈-alkoxycarbonyl, aryl or heterocyclyl radicals, and all ring systems mentioned may be substituted by 1-4 C₁-C₈-alkyl, halogen, hydroxyl, oxide, oxo, trifluoromethyl, C₁-C₈-alkoxy, C₁-C₈-alkoxy-C₁-C₈-alkyl, C₁-C₈-alkoxy-C₁-C₈-alkoxy, C₁-C₈-alkoxycarbonylamino, C₁-C₈-alkylcarbonylamino, C₁-C₈-alkylamino, N,N-di-C₁-C₈-alkylamino, aryl-C₀-C₄-alkyl, aryloxy-C₀-C₄-alkyl, aryl-C₀-C₄-alkyl-C₁-C₈-alkoxy, aryloxy-C₀-C₄-alkyl-C₁-C₈-alkoxy, heterocyclyl-C₀-C₄-alkyl, heterocyclyloxy-C₀-C₄-alkyl, heterocyclyl-C₀-C₄-alkyl-C₁-C₈-alkoxy or heterocyclyloxy-C₀-C₄-alkyl-C₁-C₈-alkoxy;
R³ is hydrogen, C₁-C₄-alkyl, C₁-C₈-alkoxycarbonyl or C₁-C₈-alkanoyl;
R⁴ is hydrogen, C₁-C₄-alkyl, C₁-C₈-alkoxycarbonyl or C₁-C₈-alkanoyl;
R⁵ are each independently hydrogen, C₁-C₈-alkyl or, together with the carbon atom to which they are bonded, are a C₃-C₈-cydoalkylidene radical; and
(A) R⁶ is a heterocyclyl radical or a polycyclic, unsaturated hydrocarbon radical which is substituted by from one to four radicals selected from C₁-C₆-alkyl, C₃₋₈-cydoalkyl, C₃₋₈-cydoalkoxy, C₃₋₈-cydoalkoxy-C₁₋₆-alkyl, C₃₋₈-cydoalkoxy-C₁₋₆-alkoxy, C₁-C₆-alkylamino, di-C₁-C₆-alkylamino, amino-C₁₋₆-alkyl, amino-C₂₋₇-alkoxy, polyhalo-C₁₋₆-alkyl, polyhalo-C₂₋₇-alkoxy, nitro, amino, C₂-C₆-alkenyl, C₁-C₆-alkoxy, C₁-C₆-alkanoyloxy, hydroxyl, halogen, oxide, oxo, cyano, carbamoyl, carboxy, C₁-C₆-alkylenedioxy, phenyl, phenoxy, phenylthio, phenyl-C₁-C₆-alkyl or phenyl-C₁-C₆-alkoxy, each of which are optionally substituted by halogen, C₁-C₆-alkyl, C₁₋₆-alkoxy, hydroxyl, C₁-C₆-alkylamino, di-C₁-C₆-alkylamino, C₁₋₆-alkoxycarbonyl, hydroxy-C₁₋₆-alkyl or trifluoromethyl, pyridylcarbonylamino-C₁₋₆-alkyl, C₂₋₇-alkenyloxy, C₁₋₆-alkoxy-C₁₋₆-alkoxy, C₁₋₆-alkoxy-C₁₋₆-alkoxy-C₁₋₆-alkyl, methoxybenzyloxy, hydroxybenzyloxy, methylenedioxybenzyloxy, dioxolanyl-C₁₋₆-alkoxy, C₃₋₈-cydoalkyl-C₁₋₆-alkyl, C₃₋₈-cycloalkyl-C₁₋₆-alkoxy, hydroxy-C₂₋₇-alkoxy, carbamoyloxy-C₂₋₇-alkoxy, pyridylcarbamoyloxy-C₂₋₇-alkoxy, benzoyloxy-C₂₋₇-alkoxy, C₁₋₆-alkoxycarbonyl, C₁₋₆-alkylcarbonylamino, C₁₋₆-alkylcarbonylamino-C₁₋₆-alkyl, C₁₋₆-alkylcarbonylamino-C₂₋₇-alkoxy, (N-C₁₋₆-alkyl)-C₁₋₆-alkylcarbonylamino-C₁₋₆-alkyl, (N-C₁₋₆-alkyl)-C₁₋₆-alkylcarbonylamino-C₂₋₇-alkoxy, C₃₋₈-cycloalkylcarbonylamino-C₁₋₆-alkyl, C₃₋₈-cycloalkylcarbonylamino-C₂₋₇-alkoxy, C₁₋₆-alkoxy-C₁₋₆-alkyl, hydroxy-C₁₋₆-alkyl, hydroxy-C₂₋₇-alkoxy-C₁₋₆-alkyl, hydroxy-C₂₋₇-alkoxy-C₁₋₆-alkoxy, C₁₋₆-alkoxycarbonylamino-C₁₋₆-alkyl, C₁₋₆-alkoxycarbonylamino-C₂₋₇-alkoxy, C₁₋₆-alkylaminocarbonylamino-C₁₋₆-alkyl, C₁₋₆-alkylaminocarbonylamino-C₂₋₇-alkoxy, C₁₋₆-alkylaminocarbonyl-C₁₋₆-alkyl, C₁₋₆-alkylaminocarbonyl-C₁₋₆-alkoxy, C₁₋₆-alkylaminocarbonyl-C₁₋₆-alkoxy-C₁₋₆-alkyl, di-C₁₋₆-alkylaminocarbonyl-C₁₋₆-alkyl, di-C₁₋₆-alkylaminocarbonyl-C₁₋₆-alkoxy, C₁₋₆-alkylcarbonyloxy-C₁₋₆-alkyl, C₁₋₆-alkylcarbonyloxy-C₂₋₆-alkoxy, cyano-C₁₋₆-alkyl, cyano-C₁₋₆-alkoxy, 2-oxooxazolidinyl-C₁₋₆-alkyl, 2-oxooxazolidinyl-C₁₋₆-alkoxy, C₁₋₆-alkoxycarbonyl-C₁₋₆-alkyl, C₁₋₆-alkoxycarbonyl-C₁₋₆-alkoxy, C₁₋₆-alkylsulphonylamino-C₁₋₆-alkyl, C₁₋₆-alkylsulphonylamino-C₂₋₇-alkoxy, (N-C₁₋₆-alkyl)-C₁₋₆-alkylsulphonylamino-C₁₋₆-alkyl, (N-C₁₋₆-alkyl)-C₁₋₆-alkylsulphonylamino-C₂₋₇-alkoxy, C₁₋₆-alkylamino-C₁₋₆-alkyl, C₁₋₆-alkylamino-C₂₋₇-alkoxy, di-C₁₋₆-alkylamino-C₁₋₆-alkyl, di-C₁₋₆-alkylamino-C₂₋₇-alkoxy, C₁₋₆-alkylsulphonyl-C₁₋₆-alkyl, C₁₋₆-alkylsulphonyl-C₁₋₆-alkoxy, carboxy-C₁₋₆-alkyl, carboxy-C₁₋₆-alkoxy, carboxy-C₁₋₆-alkoxy-C₁₋₆-alkyl, C₁₋₆-alkoxy-C₁₋₆-alkylcarbonyl, acyl-C₁₋₆-alkoxy-C₁₋₆-alkyl, (N-C₁₋₆-alkyl)-C₁₋₆-alkoxycarbonylamino, (N-hydroxy)-C₁₋₆-alkylaminocarbonyl-C₁₋₆-alkyl, (N-hydroxy)-C₁₋₆-alkylaminocarbonyl-C₁₋₆-alkoxy, (N-hydroxy)aminocarbonyl-C₁₋₆-alkyl, (N-hydroxy)aminocarbonyl-C₁₋₆-alkoxy, C₁₋₆-alkoxy-aminocarbonyl-C₁₋₆-alkyl, 6-alkoxyaminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆-alkylaminocarbonyl-C₁₋₆-alkoxy, (N-acyl)-C₁₋₆-alkoxy-C₁₋₆-alkylamino, C₁₋₆-alkoxy-C₁₋₆-alkylcarbamoyl, (N-C₁₋₆-alkyl)-C₁₋₆-alkoxy-C₁₋₆-alkylcarbamoyl, C₁₋₆-alkoxy-C₁₋₆-alkylcarbonyl, C₁₋₆-alkoxy-C₁₋₆-alkylcarbonylamino, (N-C₁₋₆-alkyl)-C₁₋₆-alkoxy-C₁₋₆-alkylcarbonylamino, 1-C₁₋₆-alkoxy-C₁₋₆-alkylimidazol-2-yl, 1-C₁₋₆-alkoxy-C₁₋₆-alkyltetrazol-5-yl, 5-C₁₋₆-alkoxy-C₁₋₆-alkyltetrazol-1-yl, 2-C₁₋₆-alkoxy-C₁₋₆-alkyl-4-oxoimidazol-1-yl, carbamoyl-C₁₋₆-alkyl, carbamoyl-C₁₋₆-alkoxy, C₁₋₆-alkylcarbamoyl, di-C₁₋₆-alkylcarbamoyl, C₁₋₆-alkylsulphonyl, C₁₋₆-alkylamidinyl, acetamidinyl-C₁₋₆-alkyl, O-methyloximyl-C₁₋₆-alkyl, O,N-dimethylhydroxylamino-C₁₋₆-alkyl, C₃₋₆-cycloalkyl-C₁₋₆-alkanoyl, aryl-C₁₋₆-alkanoyl or heterocyclyl-C₁₋₆-alkanoyl, or else pyridyl, pyridyloxy, pyridylthio, pyridylamino, pyridyl-C₁₋₆-alkyl, pyridyl-C₁₋₆-alkoxy, pyrimidinyl, pyrimidinyloxy, pyrimidinylthio, pyrimidinylamino, pyrimidinyl-C₁₋₆-alkyl, pyrimidinyl-C₁₋₆-alkoxy, thienyl, thienyl-C₁₋₆-alkyl, thienyl-C₁₋₆-alkoxy, furyl, furyl-C₁₋₆-alkyl or furyl-C₁₋₆-alkoxy, each of which is optionally substituted by halogen, C₁₋₆-alkyl, C₁₋₆-alkoxy or dihydroxy-C₁₋₆-alkylaminocarbonyl, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyl-tetrazol-1-ylalkyl, 5-methyl-tetrazol-1-ylalkoxy, thiazol-4-ylalkyl, thiazol-4-ylalkoxy, oxazol-4-ylalkyl, oxazol-4-ylalkoxy, 2-oxo-pyrrolidinylalkyl, 2-oxo-pyrrolidinylalkoxy, imidazolylalkyl, imidazolylalkoxy, 2-methyl-imidazolylalkyl, 2-methyl-imidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₆-alkoxy-C₁₋₆-alkyl-pyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl, 2-oxotetrahydropyrimidinyl and the -O-CH₂CH(OH)CH₂NRₓ radical where NRₓ is a mono- or di-C₁₋₆-alkylamino, piperidino, morpholino, piperazino or N-methylpiperazino radical; or
(B) R⁶ is a polycyclic, unsaturated hydrocarbon radical, phenyl substituted by C₁-C₆-alkylenedioxy, furyl, thienyl, pyridyl, pyrimidyl, indolyl, quinolinyl, pyrazinyl, triazolyl, imidazolyl, benzothiazolyl, pyranyl, tetrahydropyranyl, azetidinyl, morpholinyl, tetrahydroquinolinyl, tetrahydroisoquinolinyl quinazolinyl, quinoxalinyl, isoquinolyl, benzo[b]thienyl, isobenzofuranyl, benzoimidazolyl, 2-oxobenzoimidazolyl, oxazolyl, thiazolyl, pyrrolyl, pyrazolyl, triazinyl, dihydrobenzofuranyl, 2-oxodihydrobenzo[d] [1,3]oxazinyl, 4-oxodihydroimidazolyl, 5-oxo-4H[1,2,4]triazinyl, 3-oxo-4H-benzo [1,4]thiazinyl, tetrahydroquinoxalinyl, 1,1,3-trioxodihydro-2H-1λ⁶-benzo [1,4]thiazinyl, 1-oxopyridyl, dihydro-3H-benzo[1,4]oxazinyl, 3,4-dihydro-2H-benzo [1,4]oxazinyl, 2-oxotetrahydrobenzo[e][1,4]diazepinyl, 2-oxodihydrobenzo[e] [1,4]diazepinyl, 1H-pyrrolizinyl, phthalazinyl, 1-oxo-3H-isobenzofuranyl, 4-oxo-3H-thieno[2,3-d] pyrimidinyl, 3-oxo-4H-benzo[1,4]oxazinyl, [1,5]naphthyridyl, dihydro-2H-benzo [1,4]thiazinyl, 1,1-dioxodihydro-2H-benzo[1,4]thiazinyl, 2-oxo-1H-pyrido [2,3b] [1,4]oxazinyl, dihydro-1H-pyrido[2,3-b][1,4]oxazinyl, 1H-pyrrolo[2,3-b]pyridyl, benzo[1,3]dioxolyl, benzoxazolyl, 2-oxobenzooxazolyl, 2-oxo-1,3-dihydroindolyl, 2,3-dihydroindolyl, indazolyl, benzofuranyl, dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxopiperidinyl, 2-oxopyrrolidinyl, 2-oxo[1,3]oxazinyl, 2-oxoazepanyl, or 2-oxotetrahydropyrimidinyl;
for the preparation of a medication for the inhibition of beta-secretase, cathepsin D, plasmepsin II and/or HIV-protease.

2. Use according to claim 1 of a compound of the formula (la) 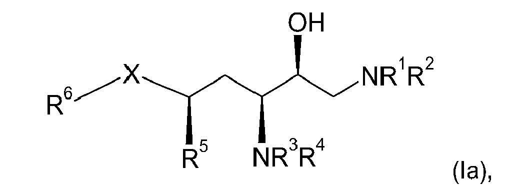 or a pharmaceutically usable salt thereof; wherein R¹, R², R³, R⁴, R⁵, R⁶ and X are each as defined in Claim 1.

3. Use according to claim 1 of a compound wherein one R⁵ radical is hydrogen and one R⁵ radical is C₁-C₈-alkyl.

4. Use according to one of claims 1 to 3 of a compound wherein R⁶ is indolyl, 3,4-dihydro-2H-benzo[1,4]oxazinyl, indazolyl, benzofuranyl, benzoimidazolyl, pyridinyl, pyrrolo[2,3-b]pyridinyl, pyrrolo[3,2-c]pyridinyl, pyrrolo[2,3-c]pyridinyl, pyrrolo[3,2-b]pyridinyl, [1,2,3]triazolo[1,5-a]pyridinyl, [1,2,4]triazolo[4,3-a]pyridinyl, imidazo[1,2-a]pyrimidinyl or imidazo[1,5-a]pyridinyl, or naphthyl, each of which is substituted by from one to four radicals selected from C₁₋₆-alkyl, cyano, oxo, oxide, trifluoromethyl, hydroxyl, halogen, carbamoyl, carboxy, C₁₋₆-alkoxy, hydroxy-C₂₋₇-alkoxy, C₁₋₆-alkoxy-C₁₋₆-alkoxy, C₁₋₆-alkoxy-C₁₋₆-alkyl or C₁₋₆-alkoxy-C₁₋₆-alkoxy-C₁₋₆-alkyl.

5. Method for the inhibition of beta-secretase, cathepsin D, plasmepsin II and/or HIV-protease consisting of the application of a therapeutically effective dose of a compound of the general formula (I) or (Ia) or a pharmaceutically usable salt thereof according to one of claims 1 to 4.

6. Use of a compound of the general formula (I) or (Ia) or a pharmaceutically usable salt thereof, according to one of claims 1 to 4 for the preparation of a medication for the prevention, delay of progression or treatment of Alzheimer Disease, malaria or HIV infection.

7. Use according to claim 6 for the preparation of a medication for the prevention, delay of progression or treatment of malaria or HIV infection..
